(19) Europäisches Patentamt
European Patent Office
Office européen des brevets

(11) EP 4 644 388 A1

(12) EUROPEAN PATENT APPLICATION
published in accordance with Art. 153(4) EPC

(43) Date of publication:
**05.11.2025 Bulletin 2025/45**

(21) Application number: **23912117.1**

(22) Date of filing: **26.12.2023**

(51) International Patent Classification (IPC):
***C07D 413/04*** *(2006.01)* ***A01N 43/88*** *(2006.01)*
***A01P 3/00*** *(2006.01)* ***C07D 231/18*** *(2006.01)*

(52) Cooperative Patent Classification (CPC):
**A01N 43/88; A01P 3/00; C07D 231/18; C07D 413/04**

(86) International application number:
**PCT/JP2023/046591**

(87) International publication number:
**WO 2024/143338 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2022 JP 2022212727**

(71) Applicant: **Nippon Soda Co., Ltd.**
**Tokyo 100-7010 (JP)**

(72) Inventors:
- **KOBAYASHI, Hiroyuki**
  **Odawara-shi, Kanagawa 250-0280 (JP)**
- **NAKAMURA, Yuka**
  **Odawara-shi, Kanagawa 250-0280 (JP)**
- **NISHIKI, Haruka**
  **Odawara-shi, Kanagawa 250-0280 (JP)**
- **SHIBAYAMA, Kotaro**
  **Odawara-shi, Kanagawa 250-0280 (JP)**
- **SAIGA, Tomoyuki**
  **Odawara-shi, Kanagawa 250-0280 (JP)**
- **SUZUKI, Hiroto**
  **Odawara-shi, Kanagawa 250-0280 (JP)**

(74) Representative: **Mewburn Ellis LLP**
**Aurora Building**
**Counterslip**
**Bristol BS1 6BX (GB)**

# (54) FIVE-MEMBERED HETEROARYL COMPOUND AND AGRICULTURAL AND HORTICULTURAL BACTERICIDE

(57) The present invention provides a compound represented by formula (I). (In the formula, the partial structure indicates a five-membered heteroaryl ring; $Q^1$ indicates a phenyl group or the like; $R^4$ indicates a hydrogen atom or the like; $R^5$ indicates a hydrogen atom, a C1-6 alkyl group, or the like; A indicates a methylene group, a dimethylene group, or the like; L indicates a single bond or the like; and $Q^2$ indicates a phenyl group or the like.) This compound has excellent bactericidal activity, is superior in terms of safety, and can be synthesized in an industrially advantageous manner.

[Chemical Formula 1]

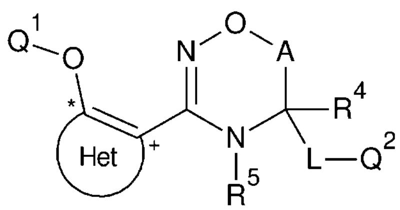

(I)

EP 4 644 388 A1

**Description**

TECHNICAL FIELD

The present invention relates to a five-membered heteroaryl compound and an agricultural and horticultural fungicide. The present invention more specifically relates to a five-membered heteroaryl compound that exhibits excellent fungicidal activity against pathogens that affect agricultural and horticultural plants, has excellent safety, and can be synthesized in an industrially advantageous manner, as well as an agricultural and horticultural fungicide containing the same as an active ingredient.

Priority is claimed on Japanese Patent Application No. 2022-212727, filed December 28, 2022, the content of which is incorporated herein by reference.

BACKGROUND ART

Various compounds that exhibit fungicidal activity against pathogens that affect agricultural and horticultural plants have been proposed. Not only sufficiently high efficacy, but also difficulty in causing drug resistance, no causing chemical damage to plants or soil pollution, and low toxicity to livestock and fish are required, so as to make such compounds to be used practically as agricultural and horticultural fungicides.

Incidentally, Patent Documents 1 and 2 disclose compounds of formula (A) and the like.

[Chemical Formula 1]

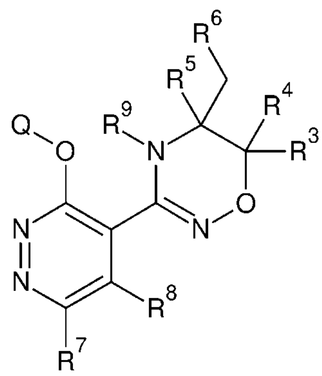

(A)

Citation List

Patent Document

Patent Document 1: Japanese Unexamined Patent Application Publication (Translation of PCT Application) No. 2022-514651

Patent Document 2: WO 2021/255071 A1

SUMMARY OF INVENTION

Technical Problem

An object of the present invention is to provide a five-membered heteroaryl compound that exhibits excellent fungicidal activity against pathogens that affect agricultural and horticultural plants, has excellent safety, and can be synthesized in an industrially advantageous manner, as well as an agricultural and horticultural fungicide containing the same as an active ingredient.

Solution to Problem

As a result of intensive research to solve the above-mentioned problems, the present invention encompassing

the following aspects has been completed.

(1) A compound of formula (I), a stereoisomer thereof, or a salt thereof.

[Chemical Formula 2]

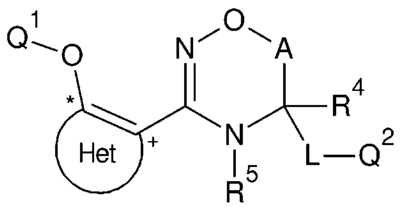

(I)

(In the formula (I),
a partial structure:

[Chemical Formula 3]

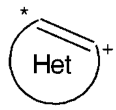

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$R^4$ is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
$R^5$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group, and
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.)

(2) The compound, stereoisomer thereof, or salt thereof, according to (1) mentioned above, in which the partial structure:

[Chemical Formula 4]

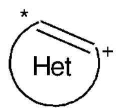

is of formula (II).

[Chemical Formula 5]

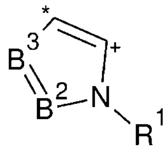

(II)

(In the formula (II),

$R^1$ is a substituted or unsubstituted C1-6 alkyl group,
$B^2$ is a nitrogen atom, or a group of formula: $CR^2$,
$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group,
$B^3$ is a nitrogen atom, or a group of formula: $CR^3$,
$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group, and
carbon atoms indicated with * and + correspond to carbon atoms indicated with * and + in the formula (I).)

(3) An agricultural and horticultural fungicide containing at least one selected from the group consisting of the compound, stereoisomer thereof, and salt thereof according to (1) or (2) mentioned above, as an active ingredient,.
(4) A method for producing a compound of formula (I-1), including
reacting a compound of formula (4) with a compound of formula (5) or a salt thereof to obtain a compound of formula (3), treating the compound of formula (3) with a halogenating agent, and then with hydroxyamine or a salt thereof to obtain a compound of formula (2), and then cyclizing the compound of formula (2) in the presence of a base to produce the compound of formula (I-1).

[Chemical Formula 6]

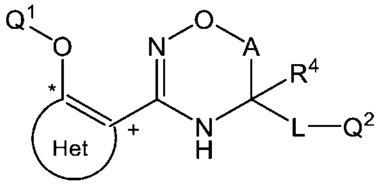

(I-1)

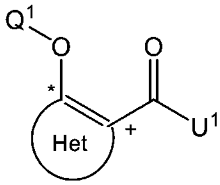

(4)

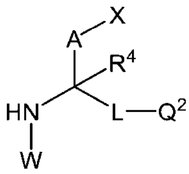

(5)

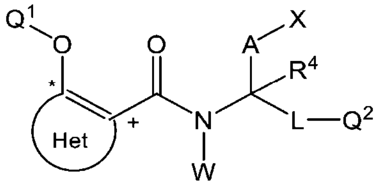

(3)

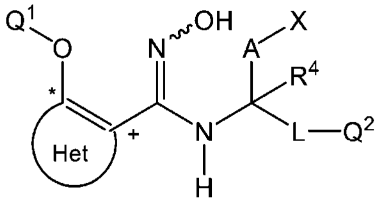

(2)

(In each formula,
the partial structure:

[Chemical Formula 7]

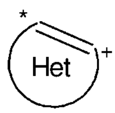

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$R^4$ is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
A is a substituted or unsubstituted methylene group, or a substituted or unsubstituted dimethylene group,
L is a single bond, a substituted or unsubstituted methylene group, or a substituted or unsubstituted dimethylene group,
$Q^2$ is a substituted or unsubstituted phenyl group, a substituted or unsubstituted naphthyl group, a substituted or

unsubstituted five- or six-membered heterocyclyl group, or a substituted or unsubstituted nine- or ten-membered heterocyclyl group,
$U^1$ is a hydroxy group or a halogeno group,
X is a halogeno group, and
W is a hydrogen atom, a t-butoxycarbonyl group, a benzyl group, an allyl group, or a (4-methoxyphenyl)methyl group.)

(5) A method for producing a compound of formula (I-2), including
reacting a compound of formula (7) with a compound of formula (8) to obtain a compound of formula (6), and treating the compound of formula (6) with a reducing agent under acidic conditions to allow cyclization to proceed, thereby producing the compound of formula (I-2).

[Chemical Formula 8]

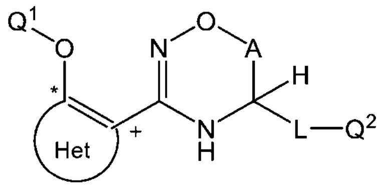

(I-2)

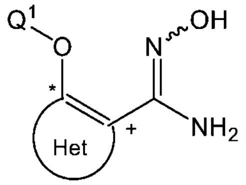

(7)

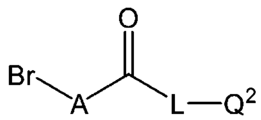

(8)

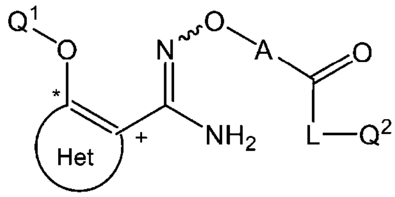

(6)

(In each formula,
a partial structure:

[Chemical Formula 9]

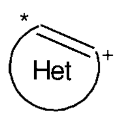

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group, and
$Q^2$ is a substituted or unsubstituted phenyl group, a substituted or unsubstituted naphthyl group, a substituted or

unsubstituted five- or six-membered heterocyclyl group, or a substituted or unsubstituted nine- or ten-membered heterocyclyl group.)

(6) A method for producing a compound of formula (I-3), including reacting a compound of formula (7) with a compound of formula (12) to obtain a compound of formula (9), treating the compound of formula (9) under oxidizing conditions with osmium trioxide and sodium periodate to allow cyclization to proceed, thereby obtaining a compound of formula (10), followed by reacting the compound of formula (10) with a compound of formula (11) under acidic conditions to produce the compound of formula (I-3).

[Chemical Formula 10]

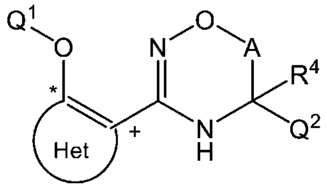

(I－3)

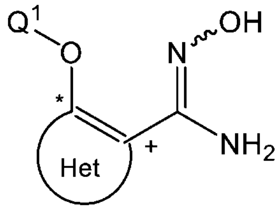

(7)

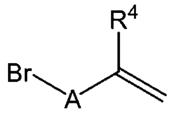

(12)

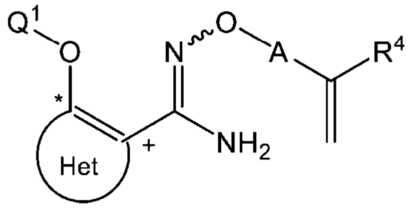

(9)

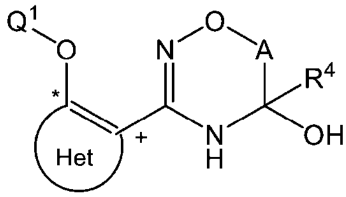

(10)

$H-Q^2$ (11)

(In each formula,
a partial structure:

[Chemical Formula 11]

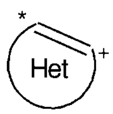

is a substituted or unsubstituted five-membered heteroaryl ring, carbon atoms indicated with * and + in the partial structure correspond to carbon atoms indicated with * and + in the formula (I),

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
A is a substituted or unsubstituted methylene group, or a substituted or unsubstituted dimethylene group, and
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.)

(7) A method for producing a compound of formula (I), including reacting a compound of formula (22) with a compound of formula (23) or a salt thereof to obtain a compound of formula (21), followed by allowing cyclization to proceed in the presence of a base, thereby producing the compound of formula (I).

[Chemical Formula 12]

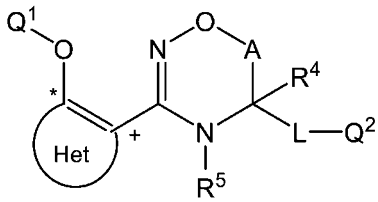

(I)

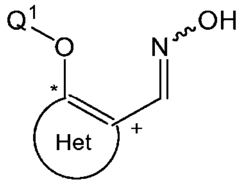

(22)

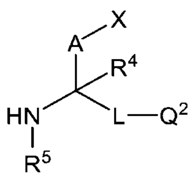

(23)

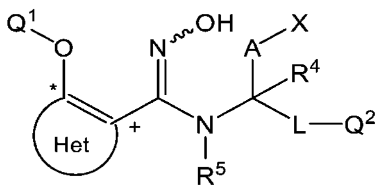

(21)

(In each formula,
a partial structure:

[Chemical Formula 13]

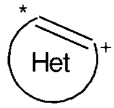

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$R^4$ is a hydrogen atom, a substituted or unsubstituted C1-6 alkyl group, a hydroxy group, a substituted or

unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
$R^5$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group, and
X is a halogeno group.)

(8) A compound of formula (7).

[Chemical Formula 14]

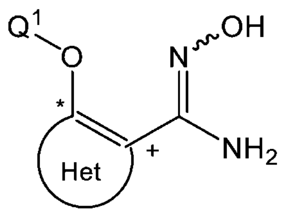

(7)

(In the formula (7),
a partial structure:

[Chemical Formula 15]

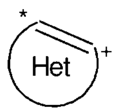

is a substituted or unsubstituted five-membered heteroaryl ring, carbon atoms indicated with * and + in the partial structure correspond to carbon atoms indicated with * and + in the formula (7), and
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.)

(9) A compound of formula (22).

[Chemical Formula 16]

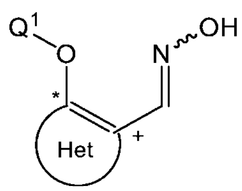

(22)

(In the formula (22),

a partial structure:

[Chemical Formula 17]

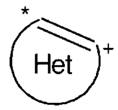

is a substituted or unsubstituted five-membered heteroaryl ring, carbon atoms indicated with * and + in the partial structure correspond to carbon atoms indicated with * and + in the formula (22), and
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group. Advantageous Effects of Invention

The five-membered heteroaryl compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively prevent diseases of agricultural and horticultural plants.

DESCRIPTION OF EMBODIMENTS

A five-membered heteroaryl compound according to the present invention is a compound of formula (I) (hereinafter, may be indicated as compound (I)), a stereoisomer of the compound (I), or a salt of the compound (I).

[Chemical Formula 18]

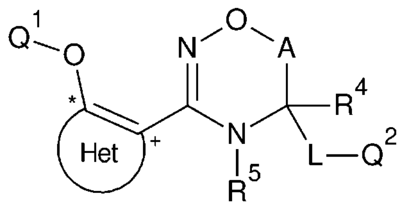

(I)

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.
On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.
The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.
There are no particular limitations on "substituents" provided that they are chemically available and achieve the effects of the present invention.
Specific examples of groups that can be "substituents" include the following groups:

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-6 cycloalkyl groups such as a cyclopropyl group, a cyclobutyl group, a cyclopentyl group, and a cyclohexyl group;
aryl groups such as a phenyl group, a tolyl group, a xylyl group, a trimethylphenyl group, and a naphthyl group;

phenyl C1-6 alkyl groups such as a benzyl group and a phenethyl group;
three- to six-membered heterocyclyl groups;
three- to six-membered heterocyclyl C1-6 alkyl groups;
a hydroxy group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
a phenoxy group, a naphthoxy group;
phenyl C1-6 alkoxy groups such as a benzyloxy group, and a phenethyloxy group;
five- or six-membered heteroaryloxy groups such as a thiazolyloxy group, and a pyridyloxy group;
five- or six-membered heteroaryl C1-6 alkyloxy groups such as a thiazolylmethyloxy group, and a pyridylmethyloxy group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
a benzoyl group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
an anilino group, a naphthylamino group;
phenyl C1-6 alkylamino groups such as a benzylamino group, and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
a phenylthio group, a naphthylthio group;
five- or six-membered heteroarylthio groups such as a thiazolylthio group, and a pyridylthio group;

C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
a phenylsulfinyl group;
five- or six-membered heteroarylsulfinyl groups such as a thiazolylsulfinyl group, and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
a phenylsulfonyl group;
five- or six-membered heteroarylsulfonyl groups such as a thiazolylsulfonyl group, and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
a triphenylsilyl group;
a cyano group; and a nitro group.

Any hydrogen atom of the "substituent" may be substituted with a group having a different structure. Examples of such a "substituent" include C1-6 alkyl groups, C1-6 haloalkyl groups, C1-6 alkoxy groups, C1-6 haloalkoxy groups, halogeno groups, a cyano group, and a nitro group.

The "three- to six-membered heterocyclyl group" contains, as a ring member atom, one to four hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "three- to six-membered heterocyclyl group" include three- to six-membered saturated heterocyclyl groups, five- or six-membered heteroaryl groups, and five- or six-membered partially-unsaturated heterocyclyl groups.

Examples of the "three- to six-membered saturated heterocyclyl groups" include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "five-membered heteroaryl groups" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "six-membered heteroaryl groups" include a pyridyl group, a pyrazinyl group, a pyrimidinyl group, a pyridazinyl group, and a triazinyl group.

Examples of the "five- or six-membered partially-unsaturated heterocyclyl groups" include: five-membered partially-unsaturated heterocyclyl groups such as a pyrrolinyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

In the formula (I), $Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.

In the formula (I), $Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.

The heterocyclyl group contains, as a ring member atom, at least one hetero atom selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings.

Examples of the "five- or six-membered heterocyclyl group" as $Q^1$ or $Q^2$ include five- or six-membered saturated heterocyclyl groups, five- or six-membered heteroaryl groups, and five- or six-membered partially-unsaturated heterocyclyl groups.

Examples of the "five- or six-membered saturated heterocyclyl group" as $Q^1$ or $Q^2$ include a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "five-membered heteroaryl group" as $Q^1$ or $Q^2$ include a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group.

Examples of the "six-membered heteroaryl group" as $Q^1$ or $Q^2$ include a pyridyl group, a pyrazinyl group, a

pyrimidinyl group, a pyridazinyl group, and a triazinyl group.

Examples of the "five- or six-membered partially-unsaturated heterocyclyl group" as $Q^1$ or $Q^2$ include: five-membered partially-unsaturated heterocyclyl groups such as a pyrrolynyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

Examples of the "nine-membered heterocyclyl group" as $Q^1$ or $Q^2$ include an indolyl group, an isoindolyl group, a benzofuranyl group, a benzothienyl group, an indazolyl group, a benzoimidazolyl group, a benzoxazolyl group, a benzoisoxazolyl group, a benzothiazolyl group, and a benzoisothiazolyl group.

Examples of the "ten-membered heterocyclyl group" as $Q^1$ or $Q^2$ include a quinolynyl group, an isoquinolynyl group, a cinnolinyl group, a phthalazinyl group, a quinazolinyl group, and a quinoxalinyl group.

Preferable examples of a substituent on the "phenyl group", "naphthyl group", "five- or six-membered heterocyclyl group" or "nine- or ten-membered heterocyclyl group" as $Q^1$ or $Q^2$ include halogeno groups, C1-6 alkyl groups, C1-6 haloalkyl groups, a hydroxy group, C1-6 alkoxy groups, C1-6 haloalkoxy groups, C3-6 cycloalkyl groups, phenyl groups, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Among them, $Q^1$ in formula (I) is preferably a substituted or unsubstituted phenyl group.

A substituent on the phenyl group as $Q^1$ is preferably at least one selected from halogeno groups, C1-6 alkyl groups, C1-6 haloalkyl groups, C1-6 haloalkoxy groups, C3-6 cycloalkyl groups, and a cyano group, and more preferably selected from halogeno groups, C1-6 alkyl groups, C1-6 haloalkoxy groups, C1-6 haloalkyl groups, and C3-6 cycloalkyl groups.

The number of substituents on the phenyl group as $Q^1$ is preferably one or two.

Among them, $Q^2$ in formula (I) is preferably a substituted or unsubstituted phenyl group, or a substituted or unsubstituted five-membered heteroaryl group, and more preferably a substituted or unsubstituted phenyl group, a substituted or unsubstituted furyl group, or a substituted or unsubstituted thienyl group.

A substituent on the phenyl group as $Q^2$ is preferably at least one selected from halogeno groups, C1-6 alkyl groups, C1-6 alkoxy groups, a phenyl group, five-membered heteroaryl groups (preferably pyrazolyl group) and a cyano group.

A substituent on the five-membered heteroaryl group as $Q^2$ is preferably at least one selected from C1-6 alkyl groups.

The number of substituents on the phenyl group or five-membered heteroaryl group as $Q^2$ is one or two.

$R^4$ in the formula (I) is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group.

The "C1-6 alkyl group" as $R^4$ may be a linear chain or a branched chain. Examples of the "C1-6 alkyl group" as $R^4$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "C1-6 alkoxy group" as $R^4$ include a methoxy group, an ethoxy group, a n-propoxy group, an i-propoxy group, a n-butoxy group, a s-butoxy group, an i-butoxy group, and a t-butoxy group.

Examples of the "C1-6 alkylcarbonyloxy group" as $R^4$ include an acetyloxy group, a propionyloxy group, a n-propylcarbonyloxy group, an i-propylcarbonyloxy group, a n-butylcarbonyloxy group, and a t-butylcarbonyloxy group.

Examples of the "C1-6 alkoxycarbonyloxy group" as $R^4$ include a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group.

Examples of the "C1-6 alkylthio group" as $R^4$ include a methylthio group, an ethylthio group, a n-propylthio group, a n-butylthio group, a n-pentylthio group, a n-hexylthio group, an i-propylthio group, and an i-butylthio group.

Examples of the "C1-6 alkylsulfinyl group" as $R^4$ include a methylsulfinyl group, an ethylsulfinyl group, and a t-butylsulfinyl group.

Examples of the "C1-6 alkylsulfonyl group" as $R^4$ include a methylsulfonyl group, an ethylsulfonyl group, and a t-butylsulfonyl group.

Preferable examples of a substituent on the "C1-6 alkyl group", "C1-6 alkoxy group", "C1-6 alkylcarbonyloxy group", "C1-6 alkoxycarbonyloxy group", "C1-6 alkylthio group", "C1-6 alkylsulfinyl group", or "C1-6 alkylsulfonyl group" as $R^4$ include halogeno groups, a hydroxy group, C1-6 alkoxy groups, C1-6 haloalkoxy groups, C3-6 cycloalkyl groups, a

phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Among them, $R^4$ in the formula (I) is preferably a hydrogen atom.

$R^5$ in the formula (I) is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group.

The "C1-6 alkyl group" as $R^5$ may be a linear chain or a branched chain. Examples of the "C1-6 alkyl group" as $R^5$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "C2-6 alkenyl group" as $R^5$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" as $R^5$ include an ethynyl group, a 1-propynyl group, a 2-propynyl group, a 1-butynyl group, a 2-butynyl group, a 3-butynyl group, a 1-methyl-2-propynyl group, a 2-methyl-3- butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkylcarbonyl group" as $R^5$ include an acetyl group, a propionyl group, a n-propylcarbonyl group, an i-propylcarbonyl group, a n-butylcarbonyl group, and a t-butylcarbonyl group.

Examples of the "C1-6 alkoxycarbonyl group" as $R^5$ include a methoxycarbonyl group, an ethoxycarbonyl group, a n-propoxycarbonyl group, an i-propoxycarbonyl group, a n-butoxycarbonyl group, and a t-butoxycarbonyl group.

Preferable examples of a substituent on the "C1-6 alkyl group", "C2-6 alkenyl group", "C2-6 alkynyl group", "C1-6 alkylcarbonyl group", or "C1-6 alkoxycarbonyl group" as $R^5$ include halogeno groups, a hydroxy group, C1-6 alkoxy groups, C1-6 haloalkoxy groups, C3-6 cycloalkyl groups, a phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Examples of the "C3-6 cycloalkyl group" as $R^5$ include a cyclopropyl group, a cyclobutyl group, a cyclopentyl group, and a cyclohexyl group.

The "five- or six-membered heterocyclyl group" as $R^5$ is a group that contains, as a ring member atom, one, two, three or four hetero atoms selected from the group consisting of a nitrogen atom, an oxygen atom and a sulfur atom. When at least two hetero atoms are contained, the hetero atoms may be identical to or different from each other.

Examples of the "five- or six-membered heterocyclyl group" include a pyrrolidinyl group, a tetrahydrofuranyl group, a thiazolidinyl group, a tetrahydro-2H-pyranyl group, a piperidyl group, a piperazinyl group, a morpholinyl group, a dioxolanyl group, a dioxanyl group; a pyrrolynyl group, a dihydrofuranyl group, an imidazolynyl group, a pyrazolynyl group, an oxazolynyl group, an isoxazolynyl group; and a dihydropyranyl group.

Preferable examples of a substituent on the "C3-6 cycloalkyl group", "phenyl group", "naphthyl group", or "five- or six-membered heterocyclyl group" as $R^5$ include halogeno groups, C 1-6 alkyl groups, C 1-6 haloalkyl groups, a hydroxy group, C 1-6 alkoxy groups, C1-6 haloalkoxy groups, C3-6 cycloalkyl groups, a phenyl group, five-membered heteroaryl groups, six-membered heteroaryl group, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Among them, $R^5$ in the formula (I) is preferably a hydrogen atom.

A in the formula (I) is a substituted or unsubstituted methylene group, or a substituted or unsubstituted dimethylene group.

L in the formula (I) is a single bond, a substituted or unsubstituted methylene group, or a substituted or unsubstituted dimethylene group.

Preferable examples of a substituent on the "methylene group" or "dimethylene group" as A or L include halogeno groups, a hydroxy group, C1-6 alkoxy groups, C1-6 haloalkoxy groups, C3-6 cycloalkyl groups, a phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Among them, A in the formula (I) is preferably a substituted or unsubstituted methylene group, and more preferably an unsubstituted methylene group.

Among them, L in the formula (I) is preferably a single bond or a substituted or unsubstituted methylene group, and more preferably a single bond or an unsubstituted methylene group.

In the formula (I), the partial structure:

[Chemical Formula 19]

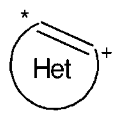

is a substituted or unsubstituted five-membered heteroaryl ring.

In the partial structure, carbon atoms indicated with * and + correspond to carbon atoms indicated with * and + in the formula (I).

Examples of the "five-membered heteroaryl ring" include a pyrrole ring, a furan ring, a thiophene ring, an imidazole ring, a pyrazole ring, an oxazole ring, an isoxazole ring, a thiazole ring, an isothiazole ring, a triazole ring, an oxadiazole ring, and a thiadiazole ring, and the five-membered heteroaryl ring is preferably a pyrazole ring or a triazole ring.

Examples of a substituent on the "five-membered heteroaryl ring" include substituted or unsubstituted C1-6 alkyl groups, substituted or unsubstituted C2-6 alkenyl groups, substituted or unsubstituted C2-6 alkynyl groups, a hydroxy group, substituted or unsubstituted C1-6 alkoxy groups, substituted or unsubstituted C1-6 alkylcarbonyl groups, substituted or unsubstituted C1-6 alkoxycarbonyl groups, substituted or unsubstituted C1-6 alkylcarbonyloxy groups, substituted or unsubstituted C1-6 alkoxycarbonyloxy groups, substituted or unsubstituted C1-6 alkylthio groups, substituted or unsubstituted C1-6 alkylsulfinyl groups, substituted or unsubstituted C1-6 alkylsulfonyl groups, substituted or unsubstituted C3-6 cycloalkyl groups, substituted or unsubstituted phenyl groups, substituted or unsubstituted naphthyl groups, substituted or unsubstituted five- or six-membered heterocyclyl groups, substituted or unsubstituted phenyloxy groups, substituted or unsubstituted naphthyloxy groups, substituted or unsubstituted five- or six-membered heterocyclyloxy groups, substituted or unsubstituted amino groups, substituted or unsubstituted aminocarbonyl groups, a nitro group, a cyano group, and halogeno groups, and a substituted or unsubstituted C1-6 alkyl group is preferable.

Examples of the "substituted or unsubstituted C1-6 alkyl groups", "substituted or unsubstituted C1-6 alkoxy groups", "substituted or unsubstituted C1-6 alkylcarbonyloxy groups", "substituted or unsubstituted C1-6 alkoxycarbonyloxy groups", "substituted or unsubstituted C1-6 alkylthio groups", "substituted or unsubstituted C1-6 alkylsulfinyl groups", or "substituted or unsubstituted C1-6 alkylsulfonyl groups" as substituents on the "five-membered heteroaryl ring" include the same groups as those mentioned as $R^4$.

Examples of the "substituted or unsubstituted C2-6 alkenyl groups", "substituted or unsubstituted C2-6 alkynyl groups", "substituted or unsubstituted C1-6 alkylcarbonyl groups", "substituted or unsubstituted C1-6 alkoxycarbonyl groups", "substituted or unsubstituted C3-6 cycloalkyl groups", "substituted or unsubstituted phenyl groups", "substituted or unsubstituted naphthyl groups", or "substituted or unsubstituted five- or six-membered heterocyclyl groups" as substituents on the "five-membered heteroaryl ring" include the same groups as those mentioned as $R^5$.

The "five- or six-membered heterocyclyloxy group" as a substituent on the "five-membered heteroaryl ring" is a group formed by substituting a hydrogen atom of a hydroxy group with a "substituted or unsubstituted five- or six-membered heterocyclyl group". Examples of the "substituted or unsubstituted five- or six-membered heterocyclyl group" include the same groups as those mentioned as $R^5$.

Preferable examples of a substituent on the "phenyloxy group", "naphthyloxy group", or "five- or six-membered heterocyclyloxy group" as a substituent on the "five-membered heteroaryl ring" include halogeno groups, C1-6 alkyl groups, C1-6 haloalkyl groups, a hydroxy group, C1-6 alkoxy groups, C1-6 haloalkoxy groups, C3-6 cycloalkyl groups, a

phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

The "amino group" as a substituent on the "five-membered heteroaryl ring" is a group of $NH_2$.

The "substituted amino group" is a group formed by substituting a hydrogen atom in a group of $NH_2$ with another group.

The "aminocarbonyl group" as a substituent on the "five-membered heteroaryl ring" is a group of $NH_2CO$.

The "substituted aminocarbonyl group" is a group formed by substituting a hydrogen atom in a group of $NH_2CO$ with another group.

Preferable examples of a substituent on the "amino group" or "aminocarbonyl group" as a substituent on the "five-membered heteroaryl ring" include C1-6 alkyl groups, C1-6 haloalkyl groups, C3-6 cycloalkyl groups, a phenyl group, five-membered heteroaryl groups, six-membered heteroaryl groups; a phenyl group substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups, and C1-6 haloalkoxy groups.

Examples of the "halogeno group" as a substituent on the "five-membered heteroaryl ring" include a fluoro group, a chloro group, a bromo group, and an iodo group.

Among them, a substituent on the "five-membered heteroaryl ring" is preferably a substituted or unsubstituted C1-6 alkyl group, or a halogeno group, and more preferably a C1-6 alkyl group, a C1-6 haloalkyl group, or a halogeno group.

In the formula (I), it is preferable that the partial structure:

[Chemical Formula 20]

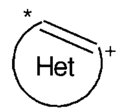

be of the formula (II).

[Chemical Formula 21]

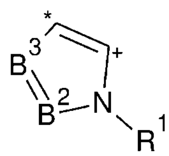

(II)

Carbon atoms indicated with * and + in the formula (II) correspond to carbon atoms indicated with * and + in the formula (I).

Namely, the five-membered heteroaryl compound according to the present invention is preferably a compound of formula (III) or a salt thereof. In the formula (III), $Q^1$, $Q^2$, $R^4$, $R^5$, A and L are the same as those in the formula (I).

[Chemical Formula 22]

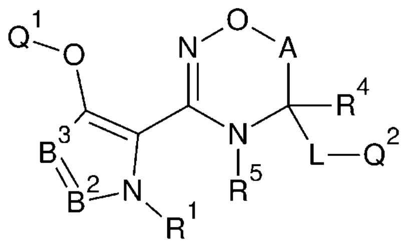

(III)

$R^1$ in the formula (II) or the formula (III) is a substituted or unsubstituted C1-6 alkyl group.

The "C1-6 alkyl group" as $R^1$ may be a linear chain or a branched chain. Examples of the "C1-6 alkyl group" as $R^1$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of a substituent on the "C1-6 alkyl group" as $R^1$ include halogeno groups such as a fluoro group, a chloro group, a bromo group, and an iodo group, and among them, a fluoro group is preferable.

In the formula the formula (II) or the formula (III), $B^2$ is a nitrogen atom or a group of formula: $CR^2$.

$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkoxy group", "substituted or unsubstituted C1-6 alkylcarbonyl group", "substituted or unsubstituted C1-6 alkoxycarbonyl group", "substituted or unsubstituted C1-6 alkylcarbonyloxy group", "substituted or unsubstituted C1-6 alkoxycarbonyloxy group", "substituted or unsubstituted C1-6 alkylthio group", "substituted or unsubstituted C1-6 alkylsulfinyl group", "substituted or unsubstituted C1-6 alkylsulfonyl group", "substituted or unsubstituted C3-6 cycloalkyl group", "substituted or unsubstituted phenyl group", "substituted or unsubstituted naphthyl group", "substituted or unsubstituted five- or six-membered heterocyclyl group", "substituted or unsubstituted phenyloxy group", "a substituted or unsubstituted naphthyloxy group", "substituted or unsubstituted five- or six-membered heterocyclyloxy group", "substituted or unsubstituted amino group", "substituted or unsubstituted aminocarbonyl group", or "halogeno group" as $R^2$ include the same as those mentioned as substituents on the "five-membered heteroaryl ring" as the partial structure.

Among them, $B^2$ in the formula (II) or the formula (III) is preferably a nitrogen atom.

In the formula (II) or the formula (III), $B^3$ is a nitrogen atom, or a group of formula: $CR^3$.

$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkoxy group", "substituted or unsubstituted C1-6 alkylcarbonyl group", "substituted or unsubstituted C1-6 alkoxycarbonyl group", "substituted or unsubstituted C1-6 alkylcarbonyloxy group", "substituted or unsubstituted C1-6 alkoxycarbonyloxy group", "substituted or unsubstituted C1-6 alkylthio group", "substituted or unsubstituted C1-6 alkylsulfinyl group", "substituted or unsubstituted C1-6 alkylsulfonyl group", "substituted or unsubstituted C3-6 cycloalkyl group", "substituted or unsubstituted phenyl group", "substituted or unsubstituted naphthyl group", "substituted or unsubstituted five- or six-membered heterocyclyl group", "substituted or unsubstituted phenyloxy group", "substituted or unsubstituted naphthyloxy group", "substituted or unsubstituted five- or six-membered heterocyclyloxy group", "substituted or unsubstituted amino group", "substituted or unsubstituted aminocarbonyl group", or "halogeno group" as $R^3$ include the same as those mentioned as $R^2$.

Among them, $R^3$ is preferably a hydrogen atom, a substituted or unsubstituted C1-6 alkyl group, or a halogeno group, more preferably a hydrogen atom or a halogeno group, and even more preferably a hydrogen atom or a chloro group.

Stereoisomers or diastereomers may exist based on an asymmetric carbon of the compound (I). Optically active forms of such isomers and mixtures of the optically active forms are encompassed in the present invention.

The salt of the compound (I) is not particularly limited as long as the salt is an agriculturally and horticulturally acceptable salt. Examples of the salt of the compound (I) include salts of inorganic acids such as hydrochloric acid and sulfuric acid; salts of organic acids such as acetic acid and lactic acid; salts of alkali metals such as lithium, sodium, and potassium; salts of alkaline earth metals such as calcium and magnesium; salts of transition metals such as iron and copper; ammonia; and salts of organic bases such as triethylamine, tributylamine, pyridine, and hydrazine.

The method for producing the compound (I) or the salt of the compound (I) is not particularly limited. For example, the compound (I) or the salt of the compound (I) can be obtained by a combination of conventionally-known chemical reactions described in the Examples, or the like. In addition, the salt of the compound (I) can be obtained from the compound (I) by a conventionally-known method.

Method for producing the compound (I)

Production method 1

A compound of formula (I-1) can be prepared by a method including a step in which a compound of formula (2) is cyclized, as shown in Scheme 1.

[Chemical Formula 23]

Scheme 1

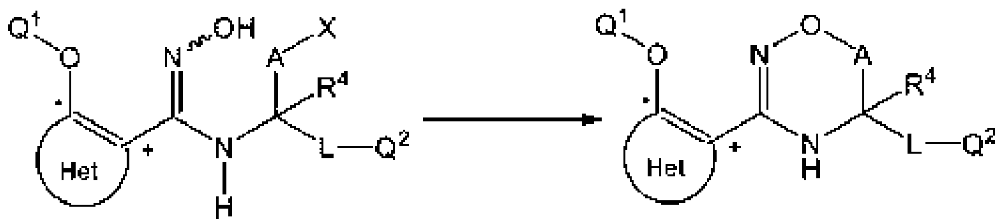

(2) (I-1)

Among the compounds (I), the compound of formula (I-1) is a compound in which $R^5$ is a hydrogen atom.

The compound of formula (2) is a compound in which X is a halogeno group, and the other symbols are the same as those defined in the compound of formula (1).

The compound of formula (2) can be produced by at least one method described in the present specification (see Production method 2).

The compound of formula (I-1) can be produced by cyclizing the compound of formula (2) in the presence of a base by a conventionally-known method.

Examples of the base include common inorganic bases and organic bases. Alkali metal carbonates, alkaline earth metal acetates, tertiary amines or aromatic amines are preferable.

Production method 2

The compound of formula (2) can be produced by a method including a step in which a compound of formula (3) is treated with a halogenating agent, followed by reacting the resultant with a hydroxyamine, as shown in Scheme 2.

[Chemical Formula24]

Scheme 2

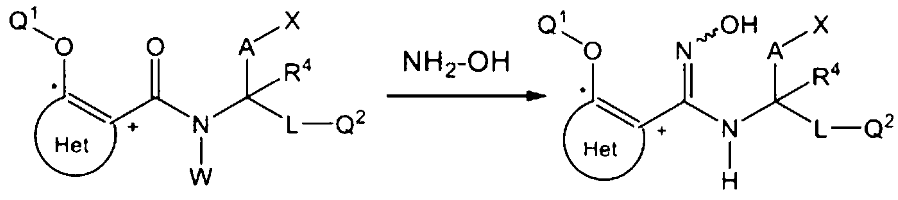


(3) (2)

The compound of formula (3) is a compound in which W is a hydrogen atom, a t-butoxycarbonyl group, a benzyl group, an allyl group, or a (4-methoxyphenyl)methyl group, X is the same as those defined in the compound of formula (2), and the other symbols are the same as those defined in the compound of formula (I).

The compound of formula (3) can be produced by at least one method described in the present specification (see Production method 3).

The compound of formula (2) can be produced by treating the compound of formula (3) with a halogenating agent by a conventionally-known method, followed by reacting the resultant with a hydroxyamine or a salt thereof.

Examples of the halogenating agent include phosgene, phosphorus tribromide, phosphorus trichloride, phosphorus pentachloride, phosphorus trichloride oxide, oxalyl chloride, and thionyl chloride.

Production method 3

The compound of formula (3) can be produced by a method including a step in which a compound of formula (4) and a compound of formula (5) or a salt thereof are reacted, as shown in Scheme 3.

[Chemical Formula 25]

Scheme 3

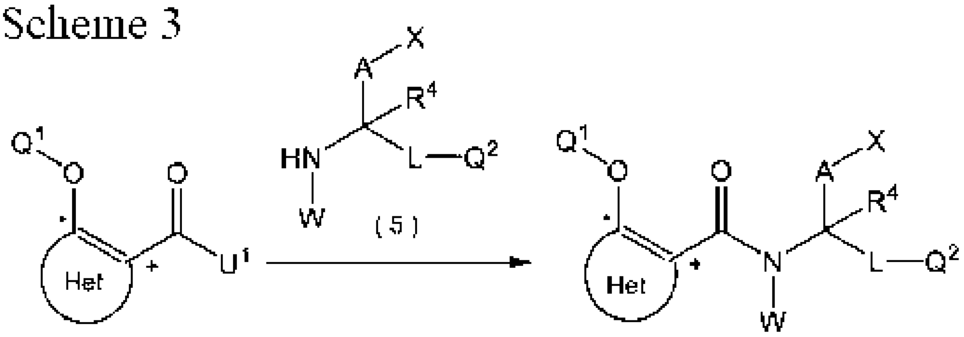

(4) (3)

The compound of formula (4) is a compound in which $U^1$ is a hydroxy group or a halogeno group, and the other symbols are the same as those defined in the compound of formula (I).

The compound of formula (5) is a compound in which X is the same as those defined in the compound of formula (2), W is the same as those defined in the compound of formula (3), and the other symbols are the same as those defined in the compound of formula (I).

The compound of formula (3) can be produced by reacting the compound of formula (4) in which $U^1$ is a hydroxy group with the compound of formula (5) or a salt thereof in the presence of a condensing agent.

Although examples of the condensing agent include halogenating agents (such as phosgene, phosphorus tribromide, phosphorus trichloride, phosphorus pentachloride, phosphorus trichloride oxide, oxalyl chloride, or thionyl chloride), dehydrating agents (such as ethyl chloroformate, methyl chloroformate, isopropyl chloroformate, isobutyl chloroformate, or methanesulfonyl chloride), and carbonyldiimidazole, the condensing agent is not limited to these.

Alternatively, the compound of formula (3) can be produced by carrying out the reaction in the presence of an

organic base and a condensing agent such as carbodiimides (such as N,N'-dicyclohexylcarbodiimide (DCC)), 1-hydroxy-7-azobenzotriazole (HOAt), O-(7-azabenzotriazol-1-yl)-N,N,N',N'-tetramethyluronium hexafluorophosphate (HATU), or propylphosphonic acid anhydride. 1-Hydroxybenzotriazole may also be used in combination.

The compound of formula (3) can be produced by reacting the compound of formula (4) in which $U^1$ is a halogeno group with the compound of formula (5) in the presence of an acid scavenger by a conventionally-known method.

Examples of the acid scavenger include common inorganic bases and organic bases. Alkali metal carbonates, alkaline earth metal acetates, tertiary amines or aromatic amines are preferable.

The compound of formula (4) may be commercially available, or may be produced by at least one method described in the present specification (see Production method 9).

The compound of formula (5) may be commercially available, or may be produced by a method described in documents (such as Japanese Unexamined Patent Application Publication (Translation of PCT Application) No. 2022-514651).

Production method 4

A compound of formula (I-2) can be produced by a method including a step in which a compound of formula (6) is treated with a reducing agent under acidic conditions to allow cyclization to proceed, as shown in Scheme 4.

[Chemical Formula 26]

Scheme 4

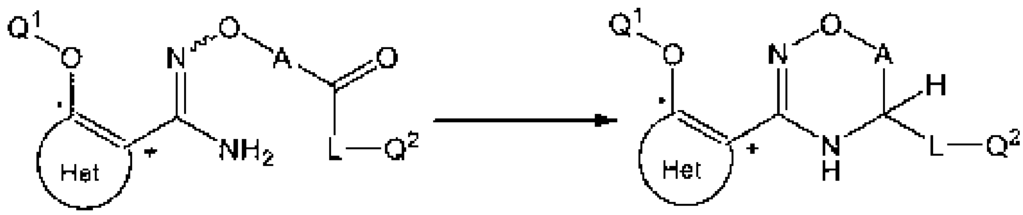

(6) (I-2)

Among the compounds (I), the compound of formula (I-2) is a compound in which $R^4$ and $R^5$ are hydrogen atoms.

The compound of formula (6) is a compound in which symbols are the same as those defined in the compound of formula (I).

The compound of formula (6) can be produced by at least one method described in the present specification (see Production method 5).

The compound of formula (I-2) can be produced by treating the compound of formula (6) under acidic conditions with a reducing agent such as sodium cyanoborohydride or sodium triacetoxyborohydride to allow cyclization to proceed. An acetic acid, trifluoroacetic acid or the like may be used to realize acidic conditions.

The reaction conditions may be referred to in the description in WO 2016/201168 A1.

Production method 5

The compound of formula (6) can be produced by a method including a step in which a compound of formula (7) and a compound of formula (8) are condensed, as shown in Scheme 5.

[Chemical Formula 27]

Scheme 5

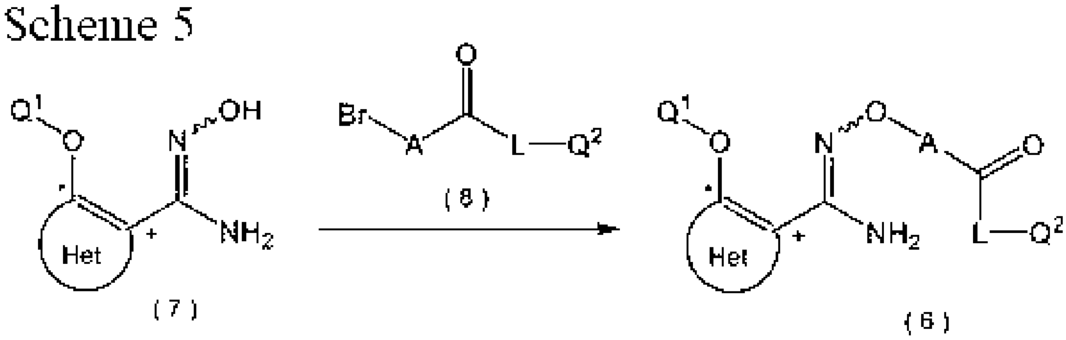

(7) (8) (6)

The compound of formula (7) is a compound in which symbols are the same as those defined in the compound of formula (I).

The compound of formula (8) is a compound in which symbols are the same as those defined in the compound of formula (I).

The compound of formula (7) can be produced by at least one method described in the present specification (see Production method 10).

The compound of formula (8) may be commercially available.

The compound of formula (6) can be produced by condensing the compound of formula (7) with the compound of formula (8) in the presence of a base by a conventionally-known method.

Examples of the base include common inorganic bases and organic bases. Alkali metal carbonates, alkaline earth metal acetates, tertiary amines or aromatic amines are preferable.

Production method 6

A compound of formula (I-3) can be produced by a method including a step in which a compound of formula (9) is treated with osmium trioxide and sodium periodate under oxidizing conditions to allow cyclization to proceed; and a step in which the resultant compound of formula (10) and a compound of formula (11) are reacted under acidic conditions, as shown in Scheme 6.

[Chemical Formula 28]

Scheme 6

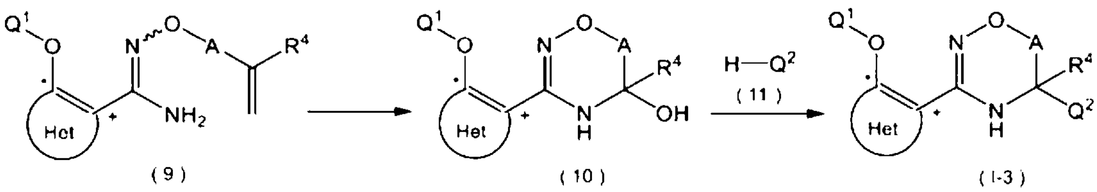

(9) (10) (11) (I-3)

Among the compounds (I), the compound of formula (I-3) is a compound in which $R^5$ is a hydrogen atom and L is a single bond.

The compound of formula (9) and the compound of formula (10) are compounds in which symbols are the same as those defined in the compound of formula (I).

The compound of formula (11) is a compound in which a symbol is the same as those defined in the compound of formula (I).

The compound of formula (9) can be produced by at least one method described in the present specification (see Production methods 7 and 8).

The compound of formula (11) may be commercially available.

The compound of formula (I-3) can be produced by condensing the compound of formula (10) with the compound of formula (11) under acidic conditions. The reaction conditions may be referred to in the description in WO 2017/031325

A1.

The compound of formula (10) can be produced by cyclizing the compound of formula (9) under oxidizing conditions, such as in the presence of osmium trioxide and sodium periodate. The reaction conditions may be referred to in the description in WO 2017/031325 A1.

Production method 7

The compound of formula (9) can be produced by a method including a step in which the compound of formula (7) and a compound of formula (12) are reacted, as shown in Scheme 7.

[Chemical Formula 29]

Scheme 7

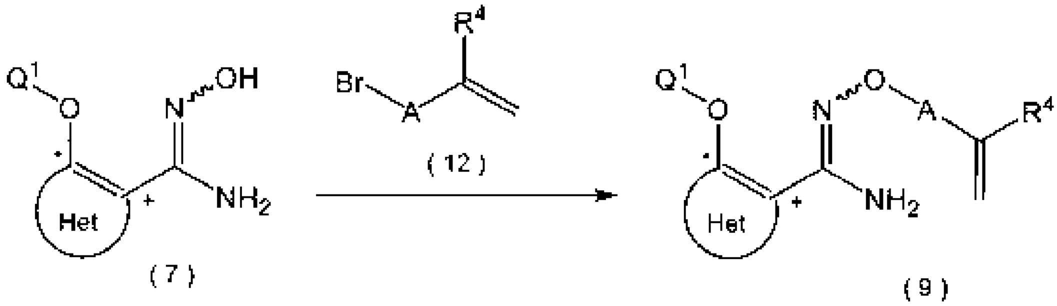

(7) (12) (9)

The compound of formula (12) is a compound in which symbols are the same as those defined in the compound of formula (1).

The compound of formula (12) may be commercially available.

The compound of formula (9) can be produced by condensing the compound of formula (7) with the compound of formula (12) in the presence of a base by a conventionally-known method.

Examples of the base include common inorganic bases and organic bases. Alkali metal carbonates, alkaline earth metal acetates, tertiary amines or aromatic amines are preferable.

Production method 8

The compound of formula (9) can be produced by a method including a step in which a compound of formula (13) and a compound of formula (14) are condensed, as shown in Scheme 8.

[Chemical Formula 30]

Scheme 8

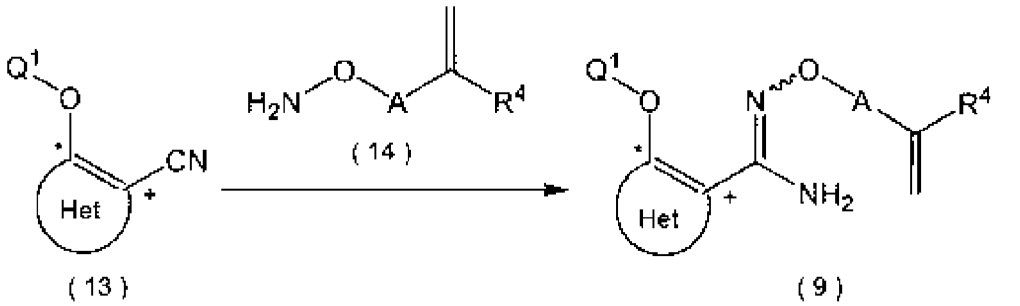

(13) (14) (9)

The compound of formula (13) is a compound in which symbols are the same as those defined in the compound of formula (I).

The compound of formula (14) is a compound in which symbols are the same as those defined in the compound of formula (I).

The compound of formula (13) may be commercially available, or may be produced by at least one method

described in the present specification (see Production method 10).

The compound of formula (14) may be commercially available.

The compound of formula (9) can be produced by condensing the compound of formula (13) with the compound of formula (14) in the presence of a base by a conventionally-known method.

Examples of the base include common inorganic bases and organic bases. Alkali metal carbonates, alkaline earth metal acetates, tertiary amines or aromatic amines are preferable.

Production method 9

The compound of formula (4) can be produced by a method including a reaction step in which the compound of formula (15) and the compound of formula (16) are reacted; and a reaction step in which the compound of formula (17) obtained by the reaction is converted to the compound of formula (4), as shown in Scheme 9.

The compound of formula (15) can be produced by a method including a reaction step in which the compound of formula (18) and a boron compound are reacted; and a reaction step in which oxidization is carried out.

[Chemical Formula 31]

Scheme 9

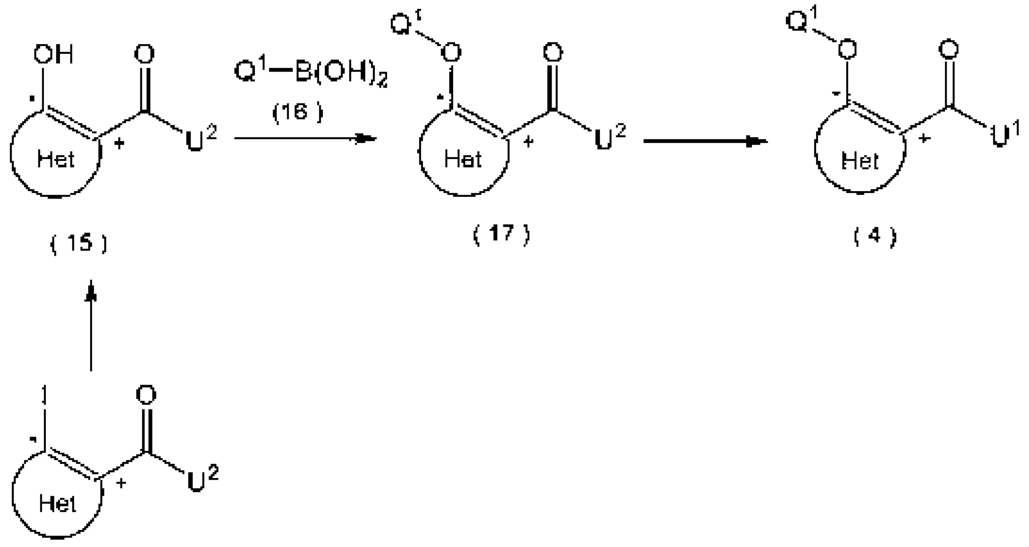


(15) (17) (4) (18)

The compound of formula (15) is a compound in which $U^2$ is a C1-6 alkoxy group and other symbols are the same as those defined in the compound of formula (I).

The compound of formula (16) is a compound in which $Q^1$ is the same as those defined in the compound of formula (I).

The compound of formula (17) is a compound in which $U^2$ is a C1-6 alkoxy group and other symbols are the same as those defined in the compound of formula (I).

The compound of formula (18) is a compound in which $U^2$ is a C1-6 alkoxy group and other symbols are the same as those defined in the compound of formula (I).

The compound of formula (17) can be produced by reacting the compound of formula (15) with the compound of formula (16) using oxygen as a reoxidant in the presence of an organic base and a copper catalyst, the so-called Chan-Lam-Evans coupling.

Although examples of the copper catalyst include divalent copper catalysts such as copper(II) chloride, copper(II) bromide, copper(II) oxide, copper(II) acetate, copper(II) sulfate, copper(II) bis(2,2,6,6-tetramethyl-3,5-heptanedioate), and copper(II) trifluoromethanesulfonate, the copper catalyst is not limited to these.

Although examples of the organic base include tertiary amines such as triethylamine, and aromatic amines such as pyridine, the organic base is not limited to these.

The compound of formula (4) in which $U^1$ is a hydroxy group can be produced by hydrolyzing the compound of formula (17) under acidic conditions or basic conditions.

For example, a method in which trifluoroacetic acid is used to realize acidic conditions, and a method in which lithium hydroxide is used in a mixed solvent system of tetrahydrofuran (THF) and water to realize basic conditions can be mentioned.

The compound of formula (4) in which $U^1$ is a halogeno group can be produced by reacting the compound of formula (4) in which $U^1$ is a hydroxy group with a halogenating agent.

The compound of formula (16) may be commercially available, or may be produced by a conventionally-known method.

The compound of formula (15) may be commercially available, or may be produced by a conventionally-known method.

Alternatively, the compound of formula (15) can be produced by reacting the compound of formula (18) with a boron compound in the presence of a palladium catalyst to convert the compound of formula (18) into an organoboron compound, followed by carrying out oxidization.

The method for converting into the organoboron compound is the so-called Miyaura-Ishiyama boronation reaction.

Although examples of the palladium catalyst include tetrakis(triphenylphosphine)palladium(0), bis(dibenzylideneacetone)palladium(0), tris(dibenzylideneacetone)dipalladium(0), bis(triphenylphosphine)palladium(II) dichloride, [1,1'-bis(diphenylphosphino)ferrocene]dichloropalladium(II), and [1,1'-bis(di-tert-butylphosphino)ferrocene]dichloropalladium(II), the palladium catalyst is not limited to these.

Although examples of the boron compound include pinacol diborane, pinacol borane, and diboronic acid, the boron compound is not limited to these.

The compound of formula (15) in which a carbon-oxygen bond is formed from a carbon-boron bond can be produced by oxidizing the obtained organoboron compound with hydrogen peroxide under basic conditions.

The compound of formula (18) may be commercially available, or may be produced by a conventionally-known method.

Production method 10

The compound of formula (7) can be produced by a method including a reaction step in which the compound of formula (19) and the compound of formula (16) are reacted; and a reaction step in which the compound of formula (13) obtained by the reaction is reacted with a hydroxyamine or a salt thereof, as shown in Scheme 10.

The compound of formula (19) may also be produced by a method including a reaction step in which the compound of formula (20) and a boron compound are reacted; and a reaction step in which oxidization is carried out.

[Chemical Formula 32]

Scheme 10

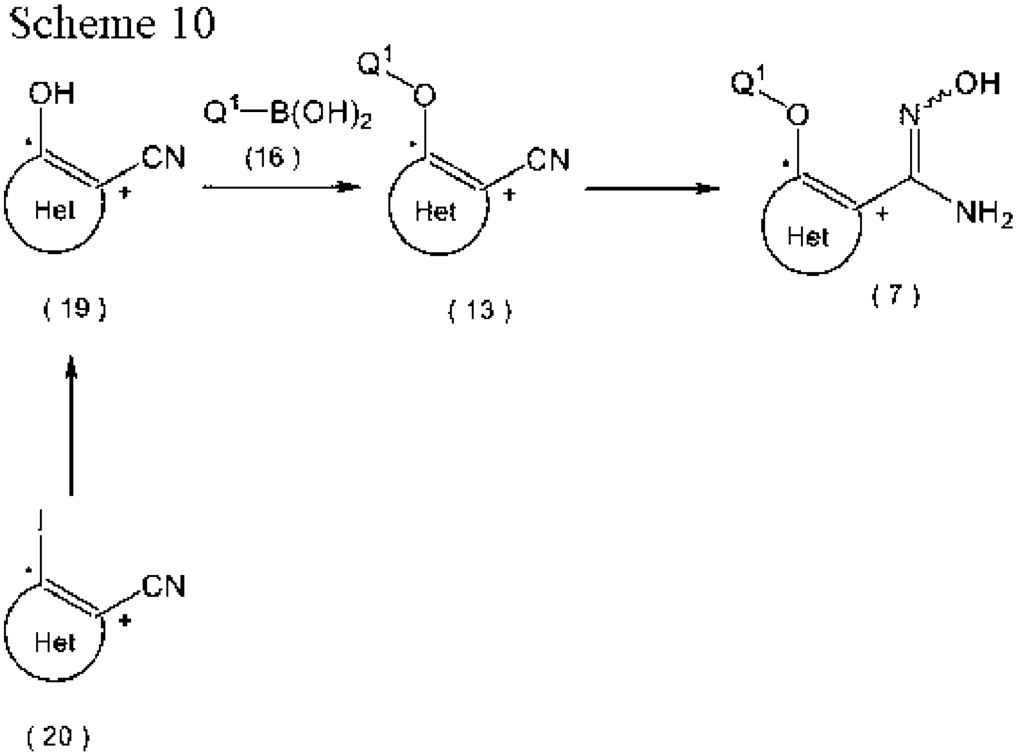


(19) (13) (7)

(20)

The compound of formula (19) is a compound in which symbols are the same as those defined in the compound of formula (I).

The compound of formula (20) is a compound in which symbols are the same as those defined in the compound of formula (I).

The compound of formula (13) can be produced by reacting the compound of formula (19) with the compound of formula (16) using oxygen as a reoxidant in the presence of an organic base and a copper catalyst, the so-called Chan-Lam-Evans coupling.

Although examples of the copper catalyst include divalent copper catalysts such as copper(II) chloride, copper(II) bromide, copper(II) oxide, copper(II) acetate, copper(II) sulfate, copper(II) bis(2,2,6,6-tetramethyl-3,5-heptanedioate), and copper(II) trifluoromethanesulfonate, the copper catalyst is not limited to these.

Although examples of the organic base include tertiary amines such as triethylamine, and aromatic amines such as pyridine, the organic base is not limited to these.

The compound of formula (7) can be produced by reacting the compound of formula (13) with a hydroxyamine or a salt thereof.

The compound of formula (19) may be commercially available, or may be produced by a conventionally-known method.

Alternatively, the compound of formula (19) can be produced by reacting the compound of formula (20) with a boron compound in the presence of a palladium catalyst to convert the compound of formula (20) into an organoboron compound, followed by carrying out oxidization.

The method for converting into the organoboron compound is the so-called Miyaura-Ishiyama boronation reaction.

Although examples of the palladium catalyst include tetrakis(triphenylphosphine)palladium(0), bis(dibenzylideneacetone)palladium(0), tris(dibenzylideneacetone)dipalladium(0), bis(triphenylphosphine)palladium(II) dichloride, [1,1'-bis(diphenylphosphino)ferrocene]dichloropalladium(II), and [1,1'-bis(di-tert-butylphosphino)ferrocene]dichloropalladium(II), the palladium catalyst is not limited to these.

Although examples of the boron compound include pinacol diborane, pinacol borane, and diboronic acid, the boron compound is not limited to these.

The compound of formula (19) in which a carbon-oxygen bond is formed from a carbon-boron bond can be produced by oxidizing the obtained organoboron compound with hydrogen peroxide under basic conditions.

The compound of formula (20) may be commercially available, or may be produced by a conventionally-known

method.

Production method 11

The compound of formula (I) can be produced by a method including a step in which a compound of formula (21) is cyclized in the presence of a base, as shown in Scheme 11.

[Chemical Formula 33]

Scheme 11

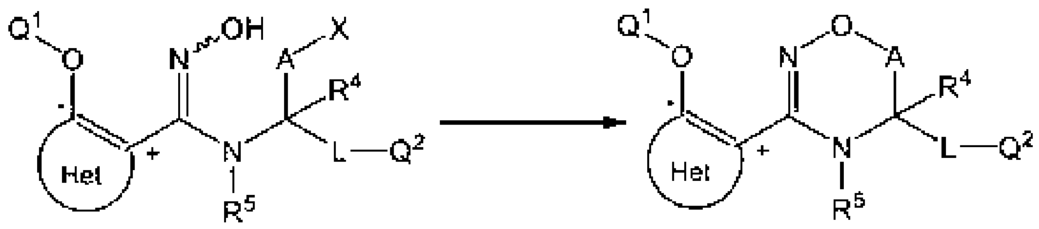

(21) (I)

The compound of formula (21) is a compound in which X is a halogeno group, and the other symbols are the same as those defined in the compound of formula (I).

The compound of formula (21) can be produced by at least one method described in the present specification (see Production method 12).

The compound of formula (I) can be produced by cyclizing the compound of formula (21) in the presence of a base by a conventionally-known method.

Examples of the base include common inorganic bases and organic bases. Alkali metal carbonates, alkaline earth metal acetates, tertiary amines or aromatic amines are preferable.

Production method 12

The compound of formula (21) can be produced by a method including a step in which a compound of formula (22) and a compound of formula (23) or a salt thereof are reacted, as shown in Scheme 12.

[Chemical Formula 34]

Scheme 12

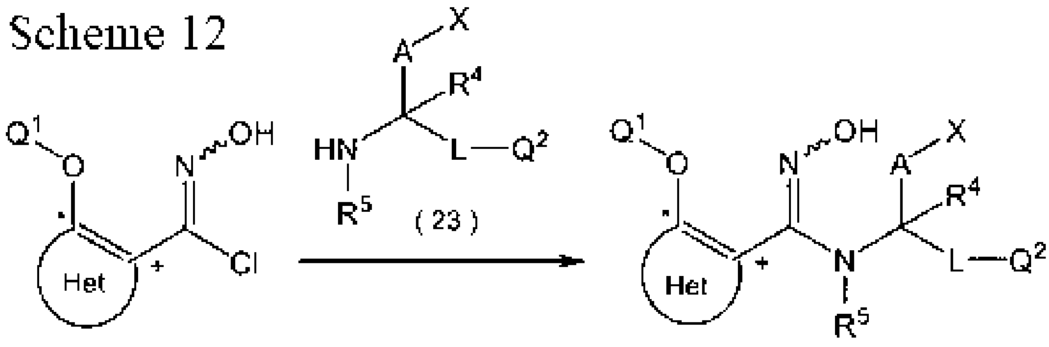


(22) (21)

The compound of formula (22) is a compound in which symbols are the same as those defined in the compound of formula (I).

The compound of formula (23) is a compound in which symbols are the same as those defined in the compound of formula (I).

The compound of formula (22) can be reacted with the compound of formula (23) in the presence of an acid scavenger so as to carry out production.

Examples of the acid scavenger include common inorganic bases and organic bases. Alkali metal carbonates, alkaline earth metal acetates, tertiary amines or aromatic amines are preferable.

The compound of formula (22) can be produced by at least one method described in the present specification (see Production method 13).

The compound of formula (23) may be commercially available, or may be produced by a method described in documents (such as Japanese Unexamined Patent Application Publication (Translation of PCT Application) No. 2022-514651).

Production method 13

The compound of formula (22) can be produced by a method including a reaction step in which the compound of formula (24) and a hydroxyamine or a salt thereof are reacted; and a reaction step in which the compound of formula (25) obtained by the reaction is treated with a halogenating agent, as shown in Scheme 13.

[Chemical Formula 35]

Scheme 13

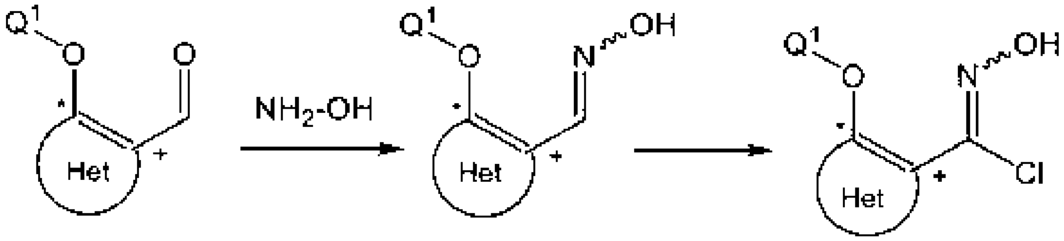


(24) (25) (22)

The compound of formula (24) is a compound in which symbols are the same as those defined in the compound of formula (I).

The compound of formula (25) is a compound in which symbols are the same as those defined in the compound of formula (I).

The compound of formula (25) can be produced by reacting the compound of formula (24) and a hydroxyamine or salt thereof.

The compound of formula (22) can be obtained by treating the compound of formula (25) with a halogenating agent such as N-chlorosuccinimide (NCS). The reaction conditions may be referred to in the description in Japanese

Unexamined Patent Application Publication (Translation of PCT Application) No. 2022-514651.

The compound of formula (24) may be commercially available, or may be produced by a conventionally-known method. For example, the compound of formula (24) may be produced by treating the compound of formula (13) with a reducing agent, or by converting the compound of formula (4) into a Weinreb amide, followed by treating the resultant with a reducing agent.

An agricultural and horticultural fungicide according to the present invention contains at least one selected from the group consisting of the compound (I), the stereoisomer of the compound (I) and the salt of the compound (I), as an active ingredient (hereinafter, may be indicated as an active ingredient (I)). The amount of the active ingredient (I) contained in the agricultural and horticultural fungicide according to the present invention is not particularly limited, as long as fungicidal effects are exhibited.

The agricultural and horticultural fungicide according to the present invention can be used to control plant diseases caused by a wide variety of filamentous fungi, such as fungi belonging to algae fungi (Oomycetes), sac fungi (Ascomycetes), imperfect fungi (Deuteromycetes), Basidiomycete fungi (Basidiomycetes) or zygomycete fungi (Zygomycetes).

Examples of plant diseases (pathogens) to be controlled include the following.

Sugar beet: brown spot disease (Cercospora beticola), black root disease (Aphanomyces cochlioides), root rot disease (Thanatephorus cucumeris), leaf rot disease (Thanatephorus cucumeris), rust disease (Uromyces betae), powdery mildew disease (Oidium sp.), spot blotch disease (Ramularia beticola), damping-off disease (Aphanomyces cochlioides, or Pythium ultimum), and the like.

Peanut: brown spot disease (Mycosphaerella arachidis), leaf mold (Ascochyta sp.), rust disease (Puccinia arachidis), damping-off disease (Pythium debaryanum), rust spot disease (Alternaria alternata), stem rot disease (Sclerotium rolfsii), black rust disease (Mycosphaerella berkeleyi), red crown rot disease (Calonectria ilicicola), and the like.

Cucumber: powdery mildew disease (Sphaerotheca fuliginea), downy mildew disease (Pseudoperonospora cubensis), gummy stem blight disease (Mycosphaerella melonis), wilt disease (Fusarium oxysporum), sclerotinia rot disease (Sclerotinia sclerotiorum), gray mold disease (Botrytis cinerea), anthracnose (Colletotrichum orbiculare), scab (Cladosporium cucumerinum), brown spot disease (Corynespora cassiicola), damping-off disease (Pythium debaryanum, Rhizoctonia solani Kuhn), Phomopsis root rot disease (Phomopsis sp.), Bacterial spot (Pseudomonas syringae pv. Lachrymans), and the like.

Tomato: gray mold disease (Botrytis cinerea), leaf mold disease (Cladosporium fulvum), late blight disease (Phytophthora infestans), verticillium wilt disease (Verticillium albo-atrum, Verticillium dahliae), powdery mildew disease (Oidium neolycopersici), early blight disease (Alternaria solani), leaf mold disease (Pseudocercospora fuligena), bacterial wilt disease (Ralstonia solanacearum), sclerotinia rot disease (Sclerotinia sclerotiorum), and the like.

Eggplant: gray mold disease (Botrytis cinerea), black rot disease (Corynespora melongenae), powdery mildew disease (Erysiphe cichoracearum), leaf mold disease (Mycovellosiella nattrassii), sclerotinia rot disease (Sclerotinia sclerotiorum), verticillium wilt disease (Verticillium dahliae), phomopsis blight disease (Phomopsis vexans), and the like.

Pepper: Phytophthora rot disease (Phytophthora capsici), gray mold disease (Botrytis cinerea), sclerotinia rot disease (Sclerotinia sclerotiorum), anthracnose (Colletotrichum aenigma, Colletotrichum capsici, Colletotrichum fructicola, Colletotrichum jiangxiense), powdery mildew disease (Leveillula taurica), and the like.

Strawberry: gray mold disease (Botrytis cinerea), powdery mildew disease (Sphaerotheca humuli), anthracnose (Colletotrichum acutatum, Colletotrichum fragariae), phytophthora rot disease (Phytophthora cactorum), soft rot disease (Rhizopus stolonifer), fusarium wilt disease (Fusarium oxysporum), verticillium wilt disease (Verticillium dahliae), sclerotinia rot disease (Sclerotinia sclerotiorum), and the like.

Onion: neck rot disease (Botrytis allii), gray mold disease (Botrytis cinerea), leaf blight disease (Botrytis squamosa), downy mildew disease (Peronospora destructor), phytophthora porri disease (Phytophthora porri), leaf blight disease (Ciobrinia allii), small sclerotial neck rot disease (Botrytis squamosa), fusarium basal rot disease (Fusarium oxysporum), pink root rot disease (Pyrenochaeta terrestris), white rot disease (Sclerotium cepivorum), rust disease (Puccinia allii), southern blight disease (Sclerotium rolfsii) and the like.

Green onion: soft rot disease (Pectobacterium carotovorum), downy mildew disease (Peronospora destructor), leaf scorch disease (Pleospora allii), bulb black rot disease (Sclerotium cepivorum), rust disease (Puccinia allii), leaf blight disease (Botrytis squamosa), southern blight disease (Sclerotium rolfsii), pink root rot disease (Pyrenochaeta terrestris), and the like.

Cabbage: clubroot disease (Plasmodiophora brassicae), soft rot disease (Erwinia carotovora), black rot disease (Xanthomonas campesrtis pv. campestris), bacterial black spot disease (Pseudomonas syringae pv. maculicala, Pseudomonas syringae pv. alisalensis), downy mildew disease (Peronospora parasitica), sclerotinia rot disease (Sclerotinia sclerotiorum), black spot disease (Alternaria brassicicola), gray mold disease (Botrytis cinerea), black leg disease (Phoma lingam), Pythium rot disease (Pythium aphanidermatum, Pythium ultimum), white rust disease (Albugo macrospora),and the like.

Lettuce: bacterial rot disease (Pseudomonas cichorii, Pseudomonas marginalis), bacterial soft rot disease (Pectobacterium carotovorum), downy mildew disease (Bremia lactucae), gray mold disease (Botrytis cinerea), stem rot disease (Sclerotinia sclerotiorum), big vein disease (Mirafiori lettuce big-vein ophiovirus), root rot disease (Fusarium oxysporum), bottom rot disease (Rhizoctonia solani), powdery mildew disease (Golovinomyces orontii), and the like.

Common bean: sclerotinia rot disease (Sclerotinia sclerotiorum), gray mold disease (Botrytis cinerea), anthracnose (Colletotrichum lindemuthianum), angular spot disease (Phaeoisariopsis griseola), and the like.

Pea: Mycosphaerella blight disease (Mycosphaerella blight), gray mold disease (Botrytis cinerea), stem rot disease (Sclerotinia sclerotiorum), powdery mildew disease (Erysiphe pisi), and the like.

Apple: powdery mildew disease (Podosphaera leucotricha), scab disease (Venturia inaequalis), Monilinia disease (Monilinia mali), black spot disease (Mycosphaerella pomi), valsa canker disease (Valsa mali), alternaria blotch disease (Alternaria mali), rust disease (Gymnosporangium yamadae), ring rot disease (Botryosphaeria berengeriana), anthracnose (Glomerella cingulata, Colletotrichum acutatum), leaf srot disease (Diplocarpon mali), fly speck disease (Zygophiala jamaicensis), Sooty blotch disease (Gloeodes pomigena), violet root rot disease (Helicobasidium mompa), white root rot disease (Rosellinia necatrix), gray mold disease (Botrytis cinerea), fire blight disease (Erwinia amylovora), silver leaf disease (Chondrostereum purpureum), crown gall disease (Rhizobium radiobacter, Rhizobium rhizogenes) and the like.

Japanese apricot: scab disease (Cladosporium carpophilum), gray mold disease (Botrytis cinerea), brown rot disease (Monilinia mumecola), sooty blotch disease (Peltaster sp.), plum pockets disease (Taphrina pruni), cylindrosporium leaf spot disease (Phloeosporella padi), and the like.

Persimmon: powdery mildew disease (Phyllactinia kakicola), anthracnose (Gloeosporium kaki), angular leaf spot (Cercospora kaki), circular leaf spot (Mycosphaerella nawae), gray mold disease (Botrytis cinerea), fly speck disease (Zygophiala jamaicensis) and the like.

Peach: brown rot disease (Monilinia fructicola, Monilia fructigena), scab disease (Cladosporium carpophilum), phomopsis rot disease (Phomopsis sp.), bacterial shot hole disease (Xanthomonas campestris pv. pruni), leaf curl disease (Taphrina deformans), anthracnose (Colletotrichum gloeosporioides), cylindrosporium leaf spot disease (Phloeosporella padi), Coriolus versicolor disease (Coriolus versicolor) and the like.

Almond: brown rot disease (Monilinia laxa), spot blotch disease (Stigmina carpophila), scab disease (Cladosporium carpophilum), red leaf spot disease (Polystigma rubrum), alternaria blotch disease (Alternaria alternata), anthracnose (Colletotrichum gloeospoides), and the like.

Yellow peach: brown rot disease (Monilinia fructicola), anthracnose (Colletotrichum acutatum), black spot disease (Alternaria sp.), Monilinia Kusanoi disease (Monilinia kusanoi), Mycosphaerella leaf spot disease (Mycosphaerella cerasella), powdery mildew disease (Podosphaera tridactyla), and the like.

Grape: gray mold disease (Botrytis cinerea), powdery mildew disease (Uncinula necator), ripe rot disease (Glomerella cingulata, Colletotrichum acutatum), downy mildew disease (Plasmopara viticola), anthracnose (Elsinoe ampelina), brown spot disease (Pseudocercospora vitis), black rot disease (Guignardia bidwellii), white rot disease (Coniella castaneicola), rust disease (Phakopsora ampelopsidis), Cottony bunch disease (the pathogen thereof has not been identified), crown gall disease (Rhizobium radiobacter, Rhizobium vitis), and the like.

Pear: scab disease (Venturia nashicola), rust disease (Gymnosporangium asiaticum), black spot disease (Alternaria kikuchiana), ring rot disease (Botryosphaeria berengeriana), powdery mildew disease (Phyllactinia mali), cytospora canker disease (Phomopsis fukushii), brown spot blotch disease (Stemphylium vesicarium), anthracnose (Glomerella cingulata), and the like.

Tea: ring spot disease (Pestalotiopsis longiseta, P. theae), anthracnose (Colletotrichum theae-sinensis), net blister blight disease (Exobasidium reticulatum), bacterial shoot blight disease (Pseudomonas syringae), blister blight disease (Exobasidium vexans), and the like.

Citrus fruits: scab disease (Elsinoe fawcetti), blue mold disease (Penicillium italicum), common green mold disease (Penicillium digitatum), gray mold disease (Botrytis cinerea), melanose disease (Diaporthe citri), canker disease (Xanthomonas campestris pv.Citri), powdery mildew disease (Oidium sp.), brown rot disease (Phytophthora citrophthora), anthracnose (Colletotrichum fioriniae), and the like.

Kiwi fruit: bacterial bud rot disease (Pseudomonas marginalis, Pseudomonas syringae, Pseudomonas viridiflava), bacterial canker disease (Pseudomonas syringae), gray mold disease (Botrytis cinerea), soft rot disease (Botryosphaeria dothidea, Diaporthe sp., Lasiodiplodia theobromae), sooty spot disease (Pseudocercospora actinidiae), and the like.

Olive: anthracnose (Colletotrichum acutatum, Colletotrichum gloeosporioides), Peacock spot (Spilocaea oleaginea), and the like.

Chestnut: anthracnose (Colletotrichum gloeosporioides), and the like.

Wheat: powdery mildew disease (Blumeria graminis f.sp. tritici), red mold disease (Gibberella zeae, Fusarium avenaceum, Fusarium culmorum, Fusarium crookwellense, Microdochium nivale), red rust disease (Puccinia recondita), yellow rust disease (Puccinia striiformis), brown snow mold disease (Pythium iwayamai), pink snow mold disease

(Monographella nivalis), eye spot disease (Pseudocercosporella herpotrichoides), leaf scorch disease (Septoria tritici), glume blotch disease (Leptosphaeria nodorum), typhulasnow blight disease (Typhula incarnata), sclerotinia snow blight disease (Myriosclerotinia borealis), damping-off disease (Gaeumannomyces graminis), ergot disease (Claviceps purpurea), stinking smut disease (Tilletia caries), loose smut disease (Ustilago nuda), blast disease (Pyricularia grisea), damping-off disease (Pythium spp., Fusarium spp., Rhizoctonia spp.), seedling blight disease (Pythium spp., Fusarium spp., Rhizoctonia spp.) and the like.

Barley: leaf spot disease (Pyrenophora graminea), net blotch disease (Pyrenophora teres), leaf blotch disease (Rhynchosporium secalis), loose smut disease (Ustilago tritici, U.nuda), damping-off disease (Pythium spp., Fusarium spp., Rhizoctonia spp.), seedling blight disease (Pythium spp., Fusarium spp., Rhizoctonia spp.) and the like.

Rice: blast disease (Pyricularia oryzae), sheath blight disease (Rhizoctonia solani), bakanae disease (Gibberella fujikuroi), brown spot disease (Cochliobolus miyabeanus), damping-off disease (Pythium graminicolum), bacterial leaf blight disease (Xanthomonas oryzae), bacterial seedling blight disease (Burkholderia plantarii), brown stripe disease (Acidovorax avenae), bacterial grain rot disease (Burkholderia glumae), Cercospora leaf spot disease (Cercospora oryzae), false smut disease (Ustilaginoidea virens), rice brown spot disease (Alternaria alternata, Curvularia intermedia), kernel discoloration of rice (Alternaria padwickii), pink coloring of rice grains (Epicoccam purpurascenns), and the like.

Tobacco: sclerotinia rot disease (Sclerotinia sclerotiorum), powdery mildew disease (Erysiphe cichoracearum), phytophthora rot disease (Phytophthora nicotianae), and the like.

Tulip: gray mold disease (Botrytis cinerea), Botrytis blight disease (Botrytis tulipae), leaf rot disease (Rhizoctonia solani), bulb rot disease (Rhizoctonia solani), bulb-coat rot disease (Rhizoctonia solani), and the like.

Rose: black spot disease (Diplocarpon rosae), powdery mildew disease (Erysiphe simulans, Podosphaera pannosa), gray mold disease (Botrytis cinerea), and the like.

Chrysanthemum: gray mold disease (Botrytis cinerea), rust disease (Puccinia horiana), downy mildew disease (Paraperonospora minor, Peronospora danica), Pythium root and stem rot disease (Pythium aphanidermatum, Pythium dissotocum, Pythium helicoides, Pythium oedochilum, Pythium sylvaticum), wilt disease (Rhizoctonia solani), Fusarium blight disease (Fusarium solani), and the like.

Gerbera: gray mold disease (Botrytis cinerea), powdery mildew disease (Podosphaera xanthii), and the like.

Lily: Botrytis blight disease (Botrytis elliptica, Pestalotiopsis sp.), gray mold disease (Botrytis cinerea), and the like.

Sunflower: downy mildew disease (Plasmopara halstedii), sclerotinia rot disease (Sclerotinia sclerotiorum), gray mold disease (Botrytis cinerea), and the like.

Bent grass: Sclerotinia snow blight disease (Sclerotinia borealis), large patch (Rhizoctonia solani), brown patch (Rhizoctonia solani), Dollar spot (Sclerotinia homoeocarpa), blast disease (Pyricularia sp.), Pythium red blight disease (Pythium aphanidermatum), anthracnose (Colletotrichum graminicola), and the like.

Orchard grass: powdery mildew disease (Erysiphe graminis), and the like.

Soybean: purple stain disease (Cercospora kikuchii), downy mildew disease (Peronospora manshurica), phytophthora rot disease (Phytophthora sojae), rust disease (Phakopsora pachyrhizi), sclerotinia rot disease (Sclerotinia sclerotiorum), anthracnose (Colletotrichum truncatum), gray mold disease (Botrytis cinerea), Sphaceloma scab (Elsinoe glycines), black spot disease (Diaporthe phaseolorum var. sojae), damping-off disease (Pythium spp., Fusarium spp., Rhizoctonia spp.), seedling blight disease (Pythium spp.,Fusarium spp.,Rhizoctonia spp.), and the like.

Potato: Phytophthora rot disease (Phytophthora infestans), early blight disease (Aleternaria solani), scurf disease (Thanatephorus cucumeris), verticillium wilt disease (Verticillium albo-atrum, V. dahliae, V. nigrescens), blackleg disease (Pectobacterium atrosepticum), soft rot disease (Pectobacterium carotovorum), gray mold disease (Botrytis cinerea), scab (Streptomyces spp.), stem rot disease (Sclerotinia sclerotiorum), and the like.

Yam: leaf mold disease (hasibu-byo) (Cylindrosporium dioscoreae), anthracnose (Colletotrichum gloeosporioides), blue mold disease (Penicillium sclerotigenum), and the like.

Sweet potato: violet root rot disease (Helicobasidium mompa), wilt disease (Fusarium oxysporum), and the like.

Taro: Phytophthora rot disease (Phytophthora colocasiae), stem rot disease (Rhizoctonia solani), and the like.

Ginger: root rot disease (Pythium ultimum, Pythium myriotylum), leaf spot disease (Phyllosticta zingiberis), and the like.

Banana: Panama disease (Fusarium oxysporum), Sigatoka disease (Mycosphaerella fijiensis, M. musicola), and the like.

Mango: anthracnose (Colletotrichum aenigma), canker disease (Xanthomonas campestris), stem-end rot disease (Diaporthe pseudophoenicicola, Lasiodiplodia theobromae, Lasiodiplodia spp., Neofusicoccum parvum, Neofusicoccum sp.), gray mold disease (Botrytis cinerea), and the like.

Rape seed: sclerotinia rot disease (Sclerotinia sclerotiorum), root rot disease (Phoma lingam), black leaf spot disease (Alternaria brassicae), powdery mildew disease (Erysiphe cruciferarum, Erysiphe cichoracearum, Oidium matthiolae), downy mildew disease (Peronospora parasitica), and the like.

Coffee: rust disease (Hemileia vastatrix), anthracnose (Colletotrichum coffeanum), brown eye spot disease

(Cercospora coffeicola), and the like.

Sugarcane: brown rust disease (Puccinia melanocephala), and the like.

Corn: zonate spot disease (Gloecercospora sorghi), rust disease (Puccinia sorghi), southern rust disease (Puccinia polysora), smut disease (Ustilago maydis), brown spot disease (Cochliobolus heterostrophus), northern leaf blight disease (Setophaeria turcica), damping-off disease (Pythium spp., Fusarium spp., Rhizoctonia spp.), seedling blight disease (Pythium spp., Fusarium spp., Rhizoctonia spp.), and the like.

Cotton: seedling blight disease (Pythium sp.), rust disease (Phakopsora gossypii), sour rot disease (Mycosphaerella areola), anthracnose (Glomerella gossypii), and the like.

Hop: downy mildew disease (Pseudoperonospora humuli), powdery mildew disease (Oidium sp., Podosphaera macularis), gray mold disease (Botrytis cinerea), and the like.

The agricultural and horticultural fungicide according to the present invention is preferably applied to cereals; vegetables; root vegetables; potatoes; trees, such as fruit-bearing trees, tea, coffee, or cacao; feed crops; lawn grasses; or plants such as cotton.

The agricultural and horticultural fungicide according to the present invention may be applied to any part of plants, such as leaves, stems, stalks, flowers, buds, fruits, seeds, sprouts, roots, tubers, tuberous roots, shoots, or slips. The agricultural and horticultural fungicide according to the present invention may also be applied to varieties, improved varieties, cultivars, mutants, hybrid bodies, or gene recombinants (GMO) of the plants.

The agricultural and horticultural fungicide according to the present invention may be used to carry out seed treatment, foliage application, soil application, or submerged application so as to control various diseases generated in agricultural and horticultural crop plants encompassing flowers, lawn grasses, and herbages.

The agricultural and horticultural fungicide according to the present invention may further contain additional components in addition to the active ingredient (I). Examples of the additional components include conventional carriers used to make formulations. Examples of other additional components include conventional fungicides, insecticides, acaricides, nematicides, soil pest control agents, plant regulatory agents, synergists, fertilizers, soil improvement agents, and animal feeds (hereinafter, may be indicated as active ingredient (II)). The agricultural and horticultural fungicide according to the present invention may exhibit synergistic effects by containing the active ingredient (I) and the active ingredient (II).

Specific examples of the active ingredient (II) which may be mixed or used with the agricultural and horticultural fungicide according to the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, ofurace;
(b) Adenosine deaminase inhibitors: bupirimate, dimethirimol, ethirimol;
(c) DNA/RNA synthesis inhibitors: hymexazol, octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, ethaboxam;
(b) Cell division inhibitors: pencycuron;
(c) Spectrin-like protein delocalization inhibitors: fluopicolide, fluopimomide.

(3) Respiration inhibitors:

(a) Complex I - NADH oxidation-reduction enzyme inhibitors: diflumetorimu, tolfenpyrad;
(b) Complex II - succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, inpyrfluxam;
(c) Complex III - ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, mandestrobin;
(d) Complex III - ubiquinol reductase Qi inhibitors: cyazofamid, amisulbrom, fenpicoxamid;
(e) Oxidative phosphorylation uncoupling agents: binapacryl, meptyldinocap, dinocap, fluazinam, ferimzone;

(f) Oxidative phosphorylation inhibitors (ATP synthase inhibitors): fentin acetate, fentin chloride, fentin hydroxide;
(g) ATP production inhibitors: silthiofam;
(h) Complex III: Qx (unknown) inhibitor of cytochrome bc1 (ubiquinone reductase): ametoctradin.

(4) Amino acid and protein synthesis inhibitors

(a) Methionine biosynthesis inhibitors: andoprim, cyprodinil, mepanipyrim, pyrimethanil;
(b) Protein synthesis inhibitors: blasticidin-S, kasugamycin, kasugamycin hydrochloride, streptomycin, oxytetracycline.

(5) Signal transduction inhibitors:

(a) Signal transduction inhibitors: quinoxyfen, proquinazid;
(b) Inhibitors of MAP / histidine kinase in osmotic signal transduction: fenpiclonil, fludioxonil, chlozolinate, iprodione, procymidone, vinclozolin.

(6) Lipids and cell membrane synthesis inhibitors:

(a) Inhibitors of phospholipid biosynthesis and methyltransferase: edifenphos, iprobenfos, pyrazophos, isoprothiolane;
(b) Lipid peroxidation agent: biphenyl, chloroneb, dicloran, quintozene, tecnazene, tolclofos-methyl, etridiazole;
(c) Agents that act on the cell membrane: iodocarb, propamocarb, propamocarb-hydrochloride, propamocarb-fosetylate, prothiocarb;
(d) Microorganisms that disrupt the cell membrane of pathogens: bacillus subtilis bacteria, bacillus subtilis strain QST713, bacillus subtilis strain FZB24, bacillus subtilis strain MBI600, bacillus subtilis strain D747, bacillus amyloliquefaciens;
(e) Agents that disrupt the cell membrane: melaleuca alternifolia (tea tree) extract.

(7) Inhibitors of sterol biosynthesis in the cell membrane:

(a) Inhibitors of demethylation at the C14 position in sterol biosynthesis: triforine, pyrifenox, pyrisoxazole, fenarimol, flurprimidol, nuarimol, imazalil, imazalil-sulphate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, mefentrifluconazole;
(b) Inhibitors of $\Delta 14$ reductase and $\Delta 8 \rightarrow \Delta 7$-isomerase in sterol biosynthesis: aldimorph, dodemorph, dodemorph acetate, fenpropimorph, tridemorph, fenpropidin, piperalin, spiroxamine;
(c) Inhibitors of 3-keto reductase in C4 demethylation in sterol biosynthesis: fenhexamid, fenpyrazamine;
(d) Inhibitors of squalene epoxidase in sterol biosynthesis: pyributicarb, naftifine, terbinafine.

(8) Cell wall synthesis inhibitors

(a) Trehalase inhibitor: validamycin;
(b) Chitin synthesis inhibitors: polyoxin, polyoxorim;
(c) Cellulose synthase inhibitors: dimethomorph, flumorph, pyrimorph, benthiavalicarb, benthiavalicarb-isopropyl, iprovalicarb, valifenalate, mandipropamid.

(9) Melanin biosynthesis inhibitors

(a) Melanin biosynthesis reductase inhibitors: phthalide, pyroquilon, tricyclazole;
(b) Melanin biosynthesis anhydrase inhibitors: carpropamid, diclocymet, fenoxanil.
(c) Melanin biosynthesis polyketide synthase inhibitors: tolprocarb.

(10) Host plant resistance inducer:

(a) Agents that act on the salicylic acid synthesis pathway: acibenzolar-S-methyl;

(b) Others: probenazole, tiadinil, isotianil, dichlobentiazox, ipfentrifluconazole, laminarin, reynoutria sachalinensis extract, phosphorous acid, phosphite.

(11) Agents, action of which is unclear: cymoxanil, fosetyl-aluminum, phosphoric acid, phosphate, tecloftalam, triazoxide, flusulfamide, diclomezine, methasulfocarb, cyflufenamid, metrafenone, pyriofenone, dodine, dodine free base, flutianil.
(12) Agents having multi-functionality: copper, copper salt, Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate (another name: iminoctadine triacetate), iminoctadine trialbesilate, anilazine, dithianon, quinomethionate, fluoroimide.
(13) Other agents: DBEDC, fluorofolpet, guazatine acetate, bis (8-quinolinolato) copper (II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophen, difenzoquat, difenzoquat·methyl sulfonate, flumetover, fosetyl-calcium, fosetyl-sodium, irumamycin, natamycin, nitrothal isopropyl, oxamocarb, pyrrolnitrin, tebufloquin, tolnifanide, zarilamid, algophase, amicarthiazol, oxathiapiprolin, fluoxapiprolin, metiram zinc, benthiazole, trichlamide, uniconazole, oxyfenthiin, picarbutrazox, dichlobentiazox, quinofumelin, thiuram, ambam, agrobacterium radiobacter, coniothyrium minitans, pseudomonas fluorescens, pseudomonas rhodesiae, talaromyces flavus, trichoderma atroviride, non-pathogenic erwinia carotovora, bacillus simplex, variovorax paradoxus, lactobacillus plantarum, florylpicoxamid, pyrapropoyne, fluindapyr, aminopyrifen, pyridachlometyl, ipflufenoquin, dipymetitrone.

Specific examples of the insecticides, acaricides, nematicides, soil insect pest control agents, and anthelmintic agents as the active ingredient (II) which may be mixed or used with the agricultural and horticultural fungicide according to the present invention are shown below.

(1) Acetylcholinesterase (AChE) inhibitors:

(a) Carbamate-based acetylcholinesterase (AChE) inhibitors: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, chloethocarb, metam-sodium, promecarb;
(b) Organophosphate-based acetylcholinesterase (AChE) inhibitors: acephate, azamethiphos, azinphos-ethyl, azinphos-methyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, Isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, sulprofos;

(2) GABAergic chloride ion channel blockers:
acetoprole, chlordane, endosulfan, ethiprole, fipronil, pyrafluoprole, pyriprole, camphechlor, heptachlor, dienochlor, flufiprole;
(3) Sodium channel modulators

(a) Pyrethroid-based sodium channel modulators:
acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ζ-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin, κ-bifenthrin, chloroprallethrin, heptafluthrin, meperfluthrin, ε-metofluthrin, momfluorothrin, ε- momfluorothrin, κ-tefluthrin, tetra methylfluthrin, bioethanomethrin;
(b) Sodium channel modulators (DDTs):
DDT, methoxychlor;

(4) Nicotinic acetylcholine receptor (nAChR) competitive modulators:
acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, flupyrimin;
(5) Nicotinic acetylcholine receptor (nAChR) allosteric modulators:
spinetoram, spinosad;
(6) Glutamate-gated chloride ion channel (GluCl) allosteric modulators:
abamectin, emamectin, emamectin-benzoate, lepimectin, milbemectin, ivermectin, selamectin, doramectin, eprinomectin, ivermectin, moxidectin, selamectin, milbemycin, milbemycin oxime, nemadectin;
(7) Juvenile hormone-like substances:
hydroprene, kinoprene, methoprene, fenoxycarb, pyriproxyfen, diofenolan, epofenonane, triprene;
(8) Other non-specific (multi-site) inhibitors:
methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam potassium salt, metam sodium salt;
(9) Chordotonal organ TRPV channel modulators:
flonicamid, pymetrozine, pyrifluquinazon, afidopiropen;
(10) Acarian growth inhibitors:
clofentezine, diflovidazin, hexythiazox, etoxazole.
(11) Insect midgut inner membrane disrupting agent derived from microorganisms:
bacillus thuringiensis subsp. Israelensis, bacillus sphaericus, bacillus thuringiensis subsp. aizawai, bacillus thuringiensis subsp. kurstaki, bacillus thuringiensis subsp. tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1 / Cry35Ab1; bacillus sphaericus.
(12) Mitochondrial ATP biosynthetic enzyme inhibitors:
diafenthiuron, azocyclotin, cyhexatin, fenbutatin oxide, propargite, tetradifon;
(13) Oxidative phosphorylation uncouplers that disrupt the proton gradient:
chlorfenapyr, DNOC, sulfluramid, binapacryl, dinobuton, dinocap;
(14) Nicotinic acetylcholine receptor (nAChR) channel blockers:
bensultap, cartap hydrochloride, nereistoxin, thiocyclam, thiosultap-sodium;
(15) Chitin biosynthesis inhibitors, type 0:
bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron;
(16) Chitin biosynthesis inhibitors, type 1:
buprofezin;
(17) Molting inhibitors:
cyromazine;
(18) Molting hormone (ecdysone) receptor agonists:
chromafenozide, halofenozide, methoxyfenozide, tebufenozide;
(19) Octopamine receptor agonists:
amitraz, demiditraz, chlordimeform;
(20) Mitochondrial electron transport system complex III inhibitors:
Hydramethylnon, acequinocyl, fluacrypyrim, bifenazate;
(21) Mitochondrial electron transport system complex I inhibitors (METI):
fenazaquin, fenpyroximate, pyridaben, pyrimidifen,tebufenpyrad, tolfenpyrad, rotenone;
(22) Voltage-dependent sodium channel blockers:
indoxacarb, metaflumizone;
(23) Acetyl CoA carboxylase inhibitors:
spirodiclofen, spiromesifen, spirotetramat, spiropidion.
(24) Mitochondrial electron transport system complex IV inhibitors:
aluminum phosphide, calcium phosphide, zinc phosphide, phosphine, cyanide, calcium cyanide, sodium cyanide, potassium cyanide;
(25) Mitochondrial electron transport system complex II inhibitors:

cyenopyrafen, cyflumetofen, pyflubumide.

(28) Ryanodine receptor modulators:

chlorantraniliprole, cyantraniliprole, cyclaniliprole, flubendiamide, cyhalodiamide, tetrachlorantraniliprole, tetraniliprole.

(29) Chordotonal organ modulator target site unspecified:

flonicamid;

(30) GABA-gated chloride ion channel allosteric modulators:

broflanilide, fluxamethamide, isocycloseram, afoxolaner, fluralaner, lotilaner, sarolaner;

(31) Other insecticides and acaricides:

azadirachtin, benzoximate, bifenazate, bromopropylate, quinomethionate, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazol-1-yl) benzonitrile (CAS: 943137-49-3), broflanilide, other metahnediamedes, steinernema carpocapsae, steinemema glaseri, pasteuria penetrans, paecilomyces tenuipes, paecilomyces fumosoroseus, beauveria bassiana, beauveria brongniartii, metarhizium anisopliae, verticillium lecanii, acynonapyr, benzpyrimoxan, flometoquin, fluhexafon, oxazosulfyl, tyclopyrazoflor.

(32) Anthelmintic agents:

(a) Benzimidazole-based: fenbendazole, albendazole, triclabendazole, oxibendazole, mebendazole, oxfendazole, parbendazole, flubendazole, febantel, netobimin, thiophanate, thiabendazole, cambendazole;
(b) Salicylanilide-based: closantel, oxyclozanide, rafoxanide, niclosamide;
(c) Substituted phenol-based: nitroxinil, nitroscanate;
(d) Pyrimidine-based: pyrantel, morantel;
(e) Imidazothiazole-based: levamisole, tetramisole;
(f) Tetrahydropyrimidine-based: praziquantel, epsiprantel;
(g) Other anthelmintic agents: cyclodiene, ryania, clorsulon, metronidazole, demiditraz, piperazine, diethylcarbamazine, dichlorophen, monepantel, tribendimidine, amidantel, thiacetarsamide, melarsomine, arsenamide.

Specific examples of plant regulatory agents which can be mixed or used with the agricultural and horticultural fungicide according to the present invention as the active ingredient (II) are shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (another name: aviglycine), aminooxyacetic acid, silver nitrate, cobalt chloride, IAA, 4-CPA, cloprop, 2,4-D, MCPB, indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyl adenine, 5-aminolevulinic acid, daminozide.

(Preparation formulation)

The agricultural and horticultural fungicide according to the present invention is not particularly limited by the dosage form thereof. Examples of the dosage form include wettable powder, emulsion, powder, granule, water-soluble agent, suspension, granular wettable powder, and tablet. The method for preparing the formulation is not particularly limited, and any conventional preparation method may be adopted depending on the dosage form.

Some preparation examples are shown below. The following preparation formulations are shown as examples, and may be modified to the extent that the modification is consistent with the gist of the present invention, but the present invention is not intended to be limited to the following preparation examples. The term "part" means "part by mass" unless particularly mentioned.

(Preparation Example 1: wettable powder)

40 parts of a five-membered heteroaryl compound according to the present invention, 53 parts of diatomaceous earth, 4 parts of higher alcohol sulfuric acid ester, and 3 parts of alkyl naphthalene sulfonate were mixed uniformly, and then finely pulverized to obtain wettable powders containing 40% by mass of the active ingredient.

(Preparation Example 2: emulsion)

30 parts of a five-membered heteroaryl compound according to the present invention, 33 parts of xylene, 30 parts of dimethylformamide, and 7 parts of polyoxyethylene alkyl allyl ether were mixed and dissolved to obtain an emulsion containing 30% by mass of the active ingredient.

(Preparation Example 3: granule)

5 parts of a five-membered heteroaryl compound according to the present invention, 40 parts of talc, 38 parts of clay, 10 parts of bentonite, and 7 parts of sodium alkyl sulfate were mixed uniformly, and then finely pulverized, followed by granulating to obtain a particle diameter of 0.5 to 1.0 mm, and thus granules containing 5% by mass of the active ingredient were obtained.

(Preparation Example 4: granule)

5 parts of a five-membered heteroaryl compound according to the present invention, 73 parts of clay, 20 parts of bentonite, 1 part of sodium dioctyl sulfosuccinate, and 1 part of potassium phosphate were uniformly mixed and then pulverized, followed by adding water thereto, and then kneading the mixture. Then, the resultant was granulated and dried to obtain granules containing 5% by mass of the active ingredient.

(Preparation Example 5: suspension)

10 parts of a five-membered heteroaryl compound according to the present invention, 4 parts of polyoxyethylene alkyl allyl ether, 2 parts of sodium polycarboxylate, 10 parts of glycerin, 0.2 parts of xanthan gum, and 73.8 parts of water were mixed, and then wet-pulverized until the particle size became 3 μm or less to obtain a suspension containing 10% by mass of the active ingredient.

(Preparation Example 6: granular wettable powder)

40 parts of a five-membered heteroaryl compound according to the present invention, 36 parts of clay, 10 parts of potassium chloride, 1 part of sodium alkylbenzene sulfonate, 8 parts of sodium lignin sulfonate, and 5 parts of formaldehyde condensate of sodium alkylbenzene sulfonate were mixed uniformly and then pulverized finely. Next, an appropriate amount of water was added thereto and the mixture was kneaded until the resultant became clayey. The clayey resultant was granulated and then dried to obtain granular wettable powders containing 40% by mass of the active ingredient.

Next, compound preparation examples are shown to explain the present invention further specifically. However, the present invention is not intended to be limited by the following examples.

(Compound preparation example 1)

5-(2,4-Dimethylbenzyl)-3-(1-methyl4-(3-(trifluoromethyl)phenoxy)-1H-pyrazol-5-yl)-5,6-dihydro-4H-1,2,4-oxadiazine (English name: 5-(2,4-dimethylbenzyl)-3-(1-methyl-4-(3-(trifluoromethyl)phenoxy)-1H-pyrazol-5-yl)-5,6-dihydro-4H-1,2,4-oxadiazine) (Compound No. a-1) was produced by the following steps.

Step 1: Conversion to 1,5-dimethyl-4-(4-nitro-3-(trifluoromethyl)phenoxy)-1H-pyrazole (English name: 1,5-dimethyl-4-(4-nitro-3-(trifluoromethyl)phenoxy)-1H-pyrazole)

[Chemical Formula 36]

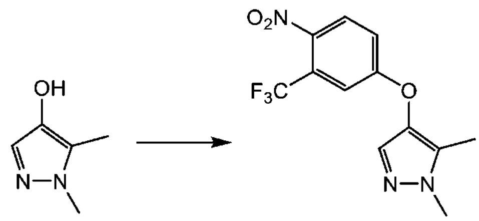

1,5-Dimethyl-1H-pyrazol-4-ol (1 g) was dissolved in dimethylformamide (DMF) (27 mL). 4-Fluoro-1-nitro-2-(trifluoromethyl)benzene (2 g) and potassium carbonate (2.5 g) were added to the resultant solution, followed by stirring the mixture for two hours at 60°C under a nitrogen atmosphere. Water was added to the resultant liquid, followed by subjecting to an extraction with ethyl acetate, and then drying over anhydrous magnesium sulfate. The solvent was distilled off under reduced pressure, and then the obtained residue was purified by silica gel column chromatography (developing solvent: n-hexane / ethyl acetate) to obtain 1.31 g of 1,5-dimethyl-4-(4-nitro-3-(trifluoromethyl)phenoxy)-1H-pyrazole (at a yield of 38%).

Step 2: Conversion to 4-((1,5-dimethyl-1H-pyrazol-4-yl)oxy)-2-trifluoromethyl)aniline (English name: 4-((1,5-dimethyl-1H-pyrazol-4-yl)oxy)-2-trifluoromethyl)aniline)

[Chemical Formula 37]

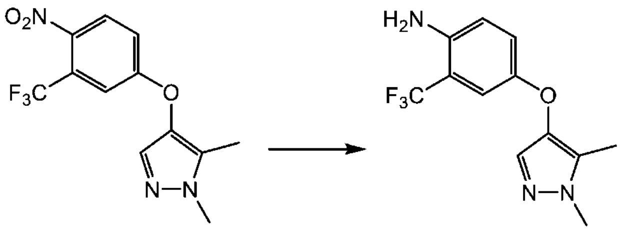

1,5-Dimethyl-4-(4-nitro-3-(trifluoromethyl)phenoxy)-1H-pyrazole (1.31 g) was dissolved in ethyl acetate (10 mL). 10% Palladium carbon (0.18 g) was added to the resultant solution, and then stirred overnight at room temperature under hydrogen atmosphere. The obtained liquid was filtered through Celite, and then the solvent was distilled off under reduced pressure. The obtained residue was purified by silica gel column chromatography modified with amino groups (developing solvent: n-hexane / ethyl acetate) to obtain 0.82 g of 4-((1,5-dimethyl-1H-pyrazol-4-yl)oxy)-2-(trifluoromethyl) aniline (at a yield of 88%).

Step 3: Conversion to 1,5-dimethyl-4-(3-(trifluoromethyl)phenoxy)-1H-pyrazole (English name: 1,5-dimethyl-4-(3-(trifluoromethyl)phenoxy)-1H-pyrazole)

[Chemical Formula 38]

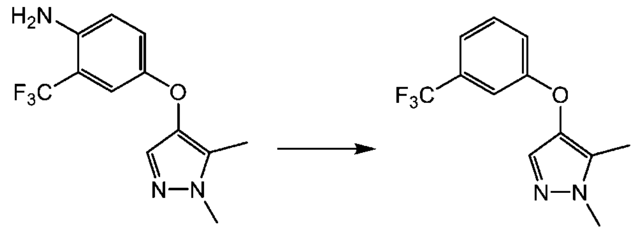

4-((1,5-Dimethyl-1H-pyrazol-4-yl)oxy)-2-(trifluoromethyl) aniline (0.82 g) was dissolved in a mixture solution composed of hydrogen chloride (3 mL) and water (1.5 mL), and then stirred at 0°C. Sodium nitrite (0.22 g) was added thereto, and then stirred at room temperature for 30 minutes. Hypophosphorous acid (0.3 g) was added dropwise gradually, and the resultant was stirred at room temperature for two hours after the end of the dropwise addition. A saturated aqueous solution of sodium bicarbonate was added to the obtained liquid, and the mixture was subjected to extraction with ethyl acetate, and then dried over anhydrous magnesium sulfate. The solvent was distilled off under reduced pressure, and the obtained residue was purified by silica gel column chromatography (developing solvent: n-hexane /ethyl acetate) to obtain 0.43 g of 1,5-dimethyl-4-(3-(trifluoromethyl)phenoxy)-1H-pyrazole (at a yield of 56%).

Step 4: Conversion to 1-methyl4-(3-(trifluoromethyl)phenoxy)-1H-pyrazole5-carboxylic acid (English name: 1-methyl-4-(3-(trifluoromethyl)phenoxy)-1H-pyrazole-5-carboxylic acid)

[Chemical Formula 39]

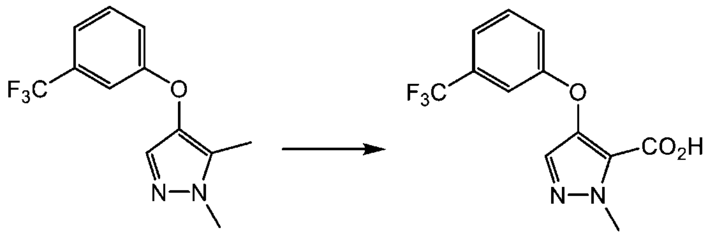

Water (20 mL) was added to 1,5-dimethyl-4-(3-(trifluoromethyl)phenoxy)-1H-pyrazole (0.43 g). Potassium permanganate (0.53 g) was added thereto, and then stirred for seven hours under heating to reflux. The obtained liquid was filtered through Celite, and then an aqueous solution containing 1M hydrochloric acid was added thereto to adjust the pH of the resultant to 1. The obtained acidic aqueous solution was extracted with ethyl acetate, and dried over anhydrous magnesium sulfate. The solvent was distilled off under reduced pressure to obtain 0.109 g of 1-methyl-4-(3-(trifluoromethyl)phenoxy)-1H-pyrazole-5-carboxylic acid (at a yield of 23%).

Step 5: Conversion to N-(1-chloro-3-(2,4-dimethylphenyl)propan-2-yl)-1-methyl-4-(3-(trifluoromethyl)phenoxy)-1H-pyrazole-5-carboxamide (English name: N-(1-chloro-3-(2,4-dimethylphenyl)propan-2-yl)-1-methyl-4-(3-(trifluoromethyl) phenoxy)-1H-pyrazole-5-carboxamide)

[Chemical Formula 40]

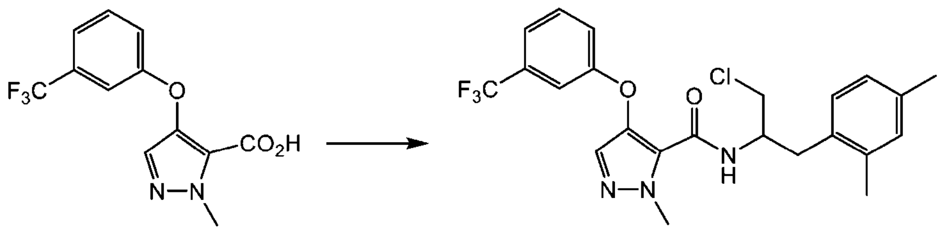

1-Methyl-4-(3-(trifluoromethyl)phenoxy)-1H-pyrazole-5-carboxylic acid (0.079 g) was added to dichloromethane (3 mL). 1-Chloro-3-(2,4-dimethylphenyl)propan-2-amine hydrochloride (0.08 g), 1-(3-dimethylaminopropyl)-3-ethylcarbodiimide hydrochloride (0.06 g), 1-hydroxybenzotriazole (0.004 g) and triethylamine (0.06 g) were added to the resultant solution and then stirred for one hour at room temperature. A saturated ammonium chloride was added to the obtained liquid, an organic layer was separated, and then the solvent was distilled off under reduced pressure. The obtained residue was purified by silica gel column chromatography (developing solvent: n-hexane / ethyl acetate) to obtain 0.11 g of N-(1-chloro3-(2,4-dimethylphenyl)propan-2-yl)-1-methyl-4-(3-(trifluoromethyl)phenoxy)-1H-pyrazole-5-carboxamide (at a yield of 86%).

Step 6: N-(1-chloro-3-(2,4-dimethylphenyl)propan-2-yl)-N'-hydroxy-1-methyl-4-(3-(trifluoromethyl)phenoxy)-1H-pyrazole-5-carboximidamide (English name: N-(1-chloro-3-(2,4-dimethylphenyl)propan-2-yl)-N'-hydroxy-1-methyl-4-(3-(trifluoromethyl)phenoxy)-1H-pyrazole-5-carboximidamide)

[Chemical Formula 41]

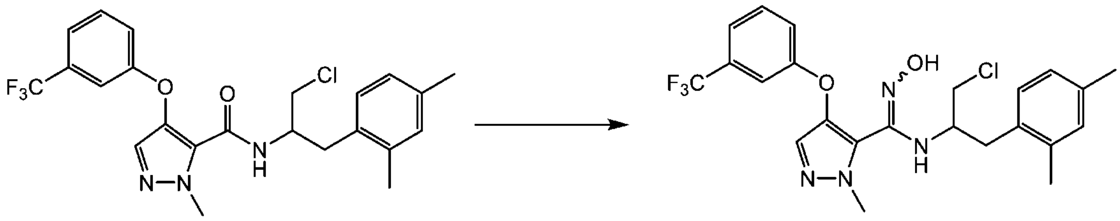

N-(1-chloro-3-(2,4-dimethylphenyl)propan-2-yl)-1-methyl-4-(3-(trifluoromethyl)phenoxy)-1H-pyrazole-5-carboxamide (0.11 g) was dissolved in toluene (3 mL). Phosphorus pentachloride (0.4 g) was added thereto, and then stirred for two hours at 80°C under a nitrogen atmosphere. The solvent was distilled off from the obtained liquid under reduced pressure. Then, toluene (3 mL) was added to the resultant, and an azeotropic mixture was distilled off. Acetonitrile (3 mL) was added to the residue, and then stirred at 0°C. Then, hydroxylamine hydrochloride (0.16 g) and N,N-diisopropylethylamine (0.62 g) were added thereto, and stirred at room temperature for 30 minutes. The obtained liquid was acidified by adding an aqueous solution containing 1M hydrochloric acid thereto, extracted with a solution of chloroform / methanol = 9/1, and then dried over anhydrous magnesium sulfate. Then, the solvent was distilled off under reduced pressure to obtain 0.09 g of N-(1-chloro3-(2,4-dimethylphenyl)propan-2-yl)-N'-hydroxy-1-methyl-4-(3-(trifluoromethyl)phenoxy)-1H-pyrazole-5-carboxamide (at a yield of 79%).

Step 7: Conversion to 5-(2,4-dimethylbenzyl)-3-(1-methyl-4-(3-(trifluoromethyl)phenoxy)-1H-pyrazol-5-yl)-5,6-dihydro-4H-1,2,4-oxadiazine (English name: 5-(2,4-dimethylbenzyl)-3-(1-methyl-4-(3-(trifluoromethyl)phenoxy)-1H-pyrazol-5-yl)-5,6-dihydro-4H-1,2,4-oxadiazine) (Compound No. a-1)

[Chemical Formula 42]

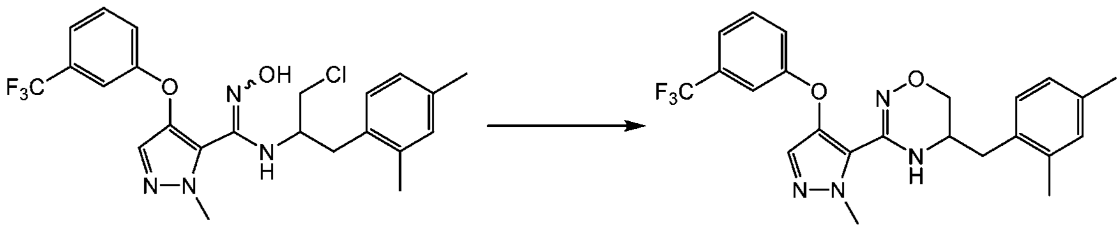

N-(1-chloro-3-(2,4-dimethylphenyl)propan-2-yl)-N'-hydroxy-1-methyl-4-(3-(trifluoromethyl)phenoxy)-1H-pyrazole-5-carboxamide (0.09 g) was dissolved in acetonitrile (5 mL), and then cesium carbonate (0.19 g) was added thereto, followed by stirring the mixture at room temperature for six hours. Water was added to the obtained liquid, and the resultant was extracted with ethyl acetate, and dried over anhydrous magnesium sulfate. Then, the solvent was distilled off under reduced pressure. The obtained residue was purified by silica gel column chromatography (developing solvent: n-hexane /ethyl acetate) to obtain 0.069 g of 5-(2,4-dimethylbenzyl)-3-(1-methyl-4-(3-(trifluoromethyl)phenoxy)-1H-pyrazol-5-yl)-5,6-dihydro-4H-1,2,4-oxadiazine (Compound No. a-1) (at a yield of 84%).

(Compound preparation example 2)

3-(1-Methyl-4-(3-(trifluoromethyl)phenoxy)-1H-pyrazol-5-yl)-5-(5-methylthiophen-2-yl)-5,6-dihydro-4H-1,2,4-oxadiazine (English name: 3-(1-methyl-4-(3-(trifluoromethyl)phenoxy)-1H-pyrazol-5-yl)-5-(5-methylthiophen-2-yl)-5,6-dihydro-4H-1,2,4-oxadiazine) (Compound No. a-26) was produced by the following steps.

Step 1: Conversion to (Z)-N'-alkoxy-1-methyl-4-(3-(trifluoromethyl)phenoxy)-1H-pyrazole-5-carboximidamide (English name: (Z)-N'-(allyloxy)-1-methyl-4-(3-(trifluoromethyl)phenoxy)-1H-pyrazole-5-carboximidamide)

[Chemical Formula 43]

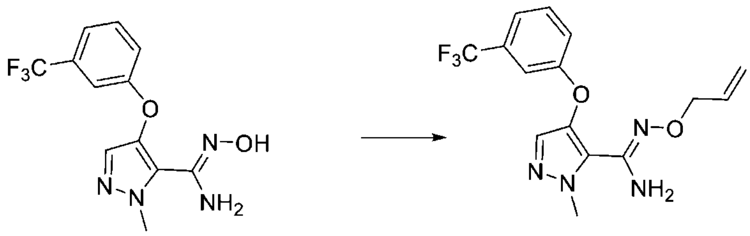

(Z)-N'-hydroxy-1-methyl-4-(3-(trifluoromethyl)phenoxy)-1H-pyrazole-5-carboximidamide (0.20 g) was dissolved in acetonitrile (3.3 mL). Cesium carbonate (0.43 g) and allyl bromide (0.085g) were added thereto, and stirred at room temperature for 18 hours.

The solvent was distilled off under reduced pressure, and the obtained residue was purified by silica gel column chromatography (developing solvent: n-hexane / ethyl acetate) to obtain 0.22 g of (Z)-N'alkoxy1-methyl-4-(3-(trifluoromethyl)phenoxy)-1H-pyrazole-5-carboximidamide (at a yield of 97%).

Step 2: Conversion to 3-(1-methyl-4-(3-(trifluoromethyl)phenoxy)-1H-pyrazol-5-yl)-5,6-dihydro-4H-1,2,4-oxadiazin-5-ol (English name: 3-(1-methyl-4-(3-(trifluoromethyl)phenoxy)-1H-pyrazol-5-yl)-5,6-dihydro-4H-1,2,4-oxadiazin-5-ol)

[Chemical Formula 44]

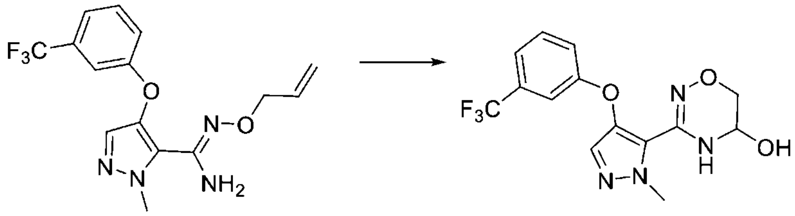

(Z)-N'alkoxy-1-methyl-4-(3-(trifluoromethyl)phenoxy)-1H-pyrazole-5-carboximidamide (0.22g) was dissolved in tetrahydrofuran (4.8 mL) and water (1.6 ml). Sodium periodate (0.41 g) and osmium tetroxide (0.033 g) were added to the resultant solution, and then stirred at room temperature for 18 hours.

An aqueous solution of sodium thiosulfate was added thereto, and the mixture was extracted with ethyl acetate, and dried over anhydrous magnesium sulfate. The solvent was distilled off under reduced pressure to obtain 0.19 g of 3-(1-methyl-4-(3-(trifluoromethyl)phenoxy)-1H-pyrazol-5-yl)-5,6-dihydro-4H-1,2,4-oxadiazin-5-ol (at a yield of 85%).

Step 3: Conversion to 3-(1-methyl-4-(3-(trifluoromethyl)phenoxy)-1H-pyrazol-5-yl)-5-(5-methylthiophen-2-yl)-5,6-dihydro-4H-1,2,4-oxadiazine (English name: 3-(1-methyl-4-(3-(trifluoromethyl)phenoxy)-1H-pyrazol-5-yl)-5-(5-methylthiophen-2-yl)-5,6-dihydro-4H-1,2,4-oxadiazine) (Compound No. a-26)

[Chemical Formula 45]

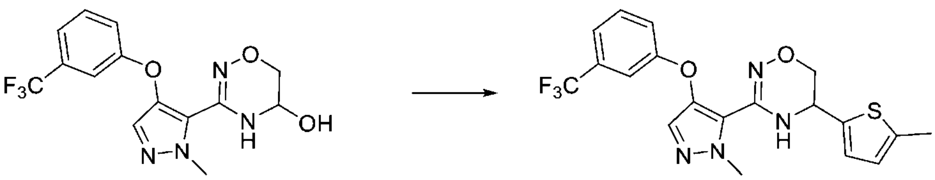

2-Methylthiophene (0.029 g) and 3-(1-methyl-4-(3-(trifluoromethyl)phenoxy)-1H-pyrazol-5-yl)-5,6-dihydro-4H-1,2,4-oxadiazin-5-ol (0.05 g) were added to formic acid (1 mL), and stirred at room temperature for 16 hours.

The solvent was distilled off under reduced pressure, and the obtained residue was purified by silica gel column chromatography (developing solvent: n-hexane /ethyl acetate) to obtain 0.025 g of 3-(1-methyl-4-(3-(trifluoromethyl)phenoxy)-1H-pyrazol-5-yl)-5-(5-methylthiophen-2-yl)-5,6-dihydro-4H-1,2,4-oxadiazine (Compound No. a-26) (at a yield of 41%).

(Compound preparation example 3)

3-(1-Methyl-4-(3-(trifluoromethyl)phenoxy)-1H-pyrazol-5-yl)-5-(thiophen-2-yl)-5,6-dihydro-4H-1,2,4-oxadiazine (English name: 3-(1-methyl-4-(3-(trifluoromethyl)phenoxy)-1H-pyrazol-5-yl)-5-(thiophen-2-yl)-5,6-dihydro-4H-1,2,4-oxadiazine) (Compound No. a-25) was produced by the following steps.

Step 1: Conversion to (Z)-N'-hydroxy-1-methyl-4-(3-(trifluoromethyl)phenoxy)-1H-pyrazole-5-carboximidamide (English name: (Z)-N'-hydroxy-1-methyl-4-(3-(trifluoromethyl)phenoxy)-1H-pyrazole-5-carboximidamide)

[Chemical Formula 46]

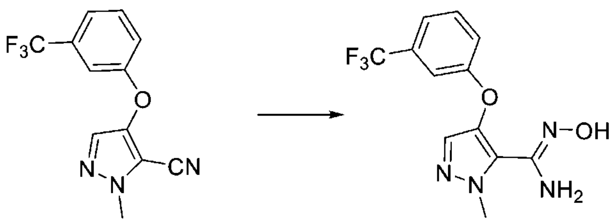

Hydroxylamine (50%wt, 0.29 g) was added to ethanol (7.4 mL), and then 1-methyl-4-(3-(trifluoromethyl)phenoxy)-1H-pyrazole-5-carbonitrile (0.59 g) was added thereto, followed by stirring the mixture at room temperature for 16 hours.

The solvent was distilled off under reduced pressure to obtain 0.63 g of (Z)-N'-hydroxy-1-methyl-4-(3-(trifluoromethyl)phenoxy)-1H-pyrazole-5-carboximidamide (at a yield of 95%).

Step 2: Conversion to (Z)-1-methyl-N'-(2-oxo-2-(thiophen-2-yl)ethoxy)-4- (3-(trifluoromethyl)phenoxy)-1H-pyrazole-5-carboximidamide (English name: (Z)-1-methyl-N'-(2-oxo-2-(thiophen-2-yl)ethoxy)-4-(3-(trifluoromethyl)phenoxy)-1H-pyrazole-5-carboximidamide)

[Chemical Formula 47]

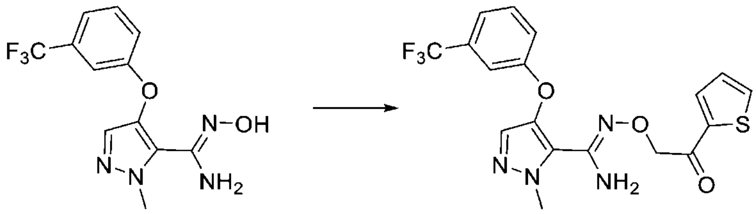

(Z)-N'-hydroxy-1-methyl-4-(3-(trifluoromethyl)phenoxy)-1H-pyrazole-5-carboximidamide (0.05g) was dissolved in acetonitrile (1.7mL). Cesium carbonate (0.11 g) and 2-bromo1-(thien-2-yl)ethan-1-one (0.051 g) were added thereto, and then stirred at room temperature for 18 hours.

The solvent was distilled off under reduced pressure, and the obtained residue was purified by silica gel column chromatography (developing solvent: n-hexane / ethyl acetate) to obtain 0.06 g of (Z)-1-methyl-N'-(2-oxo-2-(thiophen-2-yl)ethoxy)-4- (3-(trifluoromethyl)phenoxy)-1H-pyrazole-5-carboximidamide (at a yield of 80%).

Step 3: Conversion to 3-(1-methyl-4-(3-(trifluoromethyl)phenoxy)-1H-pyrazol-5-yl)-5-(thiophen-2-yl)-5,6-dihydro-4H-1,2,4-oxadiazine (English name: 3-(1-methyl-4-(3-(trifluoromethyl)phenoxy)-1H-pyrazol-5-yl)-5-(thiophen-2-yl)-5,6-dihydro-4H-1,2,4-oxadiazine) (Compound No. a-25)

[Chemical Formula 48]

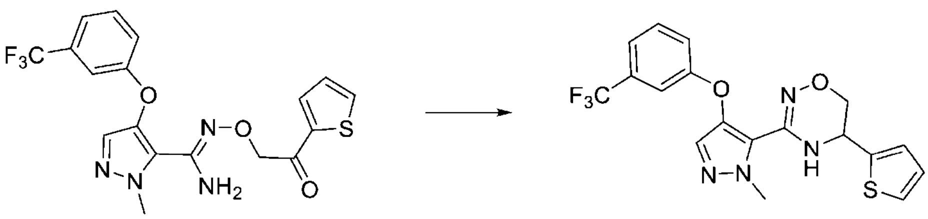

Acetic acid (0.24 ml) was added to methanol (1.2 mL), and then (Z)-1-methyl-N'-(2-oxo-2-(thiophen-2-yl)ethoxy)-4-(3-(trifluoromethyl)phenoxy)-1H-pyrazole-5-carboximidamide (0.06 g) was added thereto, followed by stirring the mixture at room temperature for four hours and then at 60°C for four hours. Then, sodium cyanoborohydride (0.018 g) was added to the resultant, followed by stirring the mixture at room temperature for four hours and then at 60°C for four hours.

The solvent was distilled off under reduced pressure, and the obtained residue was purified by silica gel column chromatography (developing solvent: n-hexane / ethyl acetate) to obtain 0.026 g of 3-(1-methyl-4-(3-(trifluoromethyl)phenoxy)-1H-pyrazol-5-yl)-5-(thiophen-2-yl)-5,6-dihydro-4H-1,2,4-oxadiazine (Compound No. a-25) (at a yield of 23%).

(Compound preparation example 4)

3-(3-Chloro-1-ethyl-4-(3-(trifluoromethyl)phenoxy)-1H-pyrazol-5-yl)-5-(2,4-dimethylbenzyl)-5,6-dihydro-4H-1,2,4-oxadiazine (English name: 3-(3-chloro-1-ethyl-4-(3-(trifluoromethyl)phenoxy)-1H-pyrazol-5-yl)-5-(2,4-dimethylbenzyl)-5,6-dihydro-4H-1,2,4-oxadiazine) (Compound No. a-30) was produced by the following steps.

Step 1: Conversion to 3-chloro-1-ethyl-4-(3-(trifluoromethyl)phenoxy)-1H-pyrazole-5-carbaldehyde (English name: 3-chloro-1-ethyl-4-(3-(trifluoromethyl)phenoxy)-1H-pyrazole-5-carbaldehyde)

[Chemical Formula 49]

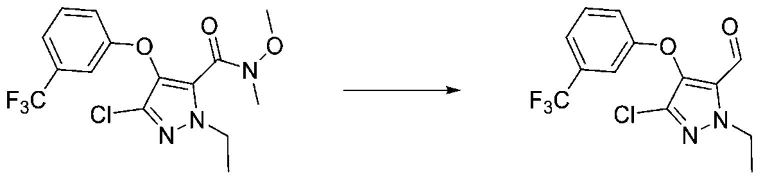

3-Chloro-1-ethyl-N-methoxy-N-methyl-4-(3-(trifluoromethyl)phenoxy)-1H-pyrazole-5- carboxamide (0.16 g) was dissolved in tetrahydrofuran (1.7 mL). Diisobutylaluminum hydride (0.14 g) was added thereto at 0°C, followed by stirring the mixture at 0°C for three hours.

Water was added to the reaction solution, and the resultant was extracted with ethyl acetate and dried over anhydrous magnesium sulfate. The solvent was distilled off under reduced pressure, and the obtained residue was purified by silica gel column chromatography (developing solvent: n-hexane / ethyl acetate) to obtain 0.11 g of 3-chloro-1-ethyl-4-(3-(trifluoromethyl)phenoxy)-1H-pyrazole-5-carbaldehyde (at a yield of 100%).

Step 2: Conversion to (E)-3-chloro-1-ethyl-4-(3-(trifluoromethyl)phenoxy)-1H-pyrazole-5-carbaldehyde oxime (English name: (E)-3-chloro-1-ethyl-4-(3-(trifluoromethyl)phenoxy)-1H-pyrazole-5-carbaldehyde oxime)

[Chemical Formula 50]

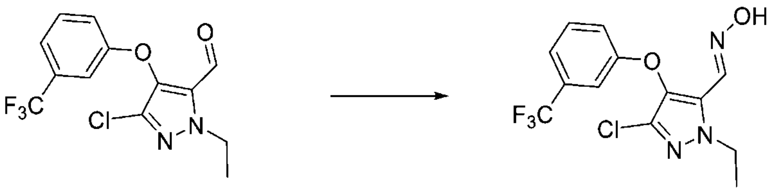

3-Chloro-1-ethyl-4-(3-(trifluoromethyl)phenoxy)-1H-pyrazole-5-carbaldehyde (0.11 g) was dissolved in pyridine(1.1 mL). Hydroxylamine hydrochloride (0.12 g) was added thereto, followed by stirring the mixture at room temperature for 24 hours.

The solvent was distilled off under reduced pressure, and the obtained residue was purified by silica gel column chromatography (developing solvent: n-hexane / ethyl acetate) to obtain 0.05 g of (E)-3-chloro-1-ethyl-4-(3-(trifluoromethyl)phenoxy)-1H-pyrazole-5-carbaldehyde oxime (at a yield of 40%).

Step 3: Conversion to 3-(3-chloro-1-ethyl-4-(3-(trifluoromethyl)phenoxy)-1H-pyrazol-5-yl)-5-(2,4-dimethylbenzyl)-5,6-dihydro-4H-1,2,4-oxadiazine (English name: 3-(3-chloro-1-ethyl-4-(3-(trifluoromethyl)phenoxy)-1H-pyrazol-5-yl)-5-(2,4-dimethylbenzyl)-5,6-dihydro-4H-1,2,4-oxadiazine) (Compound No. a-30)

[Chemical Formula 51]

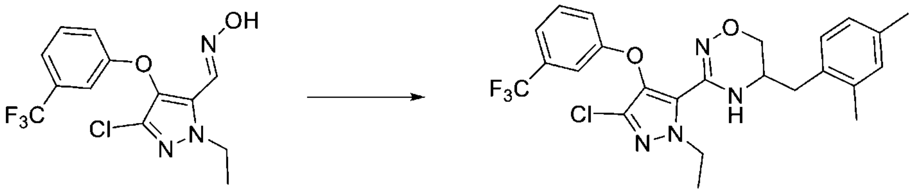

(E)-3-chloro-1-ethyl-4-(3-(trifluoromethyl)phenoxy)-1H-pyrazole-5-carbaldehyde oxime (0.05 g) was dissolved in acetonitrile (0.75 mL), and then N-chlorosuccinimide (0.024 g) was added thereto, followed by stirring the mixture at room temperature for 16 hours. Then, 1-chloro-3-(2,4-dimethylphenyl)propan-2-amine hydrochloride (0.042 g) and triethylamine (0.036 g) were added thereto, and then stirred at room temperature for three hours, followed by adding cesium carbonate (0.24 g) thereto, and then stirring the mixture at room temperature for six hours.

The solvent was distilled off under reduced pressure, and the obtained residue was purified by silica gel column chromatography (developing solvent: n-hexane / ethyl acetate) to obtain 0.026 g of 3-(3-chloro-1-ethyl-4-(3-(trifluoromethyl)phenoxy)-1H-pyrazol-5-yl)-5-(2,4-dimethylbenzyl)-5,6-dihydro-4H-1,2,4-oxadiazine (Compound No. a-30) (at a yield of 35%).

(Compound preparation example 5)

5-(2,4-Dimethylbenzyl)-3-(4-(3-ethylphenoxy)-1-methyl-1H-pyrazol-5-yl)-5,6-dihydro-4H-1,2,4-oxadiazine (English name: 5-(2,4-dimethylbenzyl)-3-(4-(3-ethylphenoxy)-1-methyl-1H-pyrazol-5-yl)-5,6-dihydro-4H-1,2,4-oxadiazine) (Compound No. a-10) was produced by the following steps.

Step 1: Conversion to methyl 1-methyl-4-(4,4,5,5-tetramethyl-1,3,2-dioxaborolan-2-yl)-1H-pyrazole-5-carboxylate (English name: methyl 1-methyl-4-(4,4,5,5-tetramethyl-1,3,2-dioxaborolan-2-yl)-1H-pyrazole-5-carboxylate)

[Chemical Formula 52]

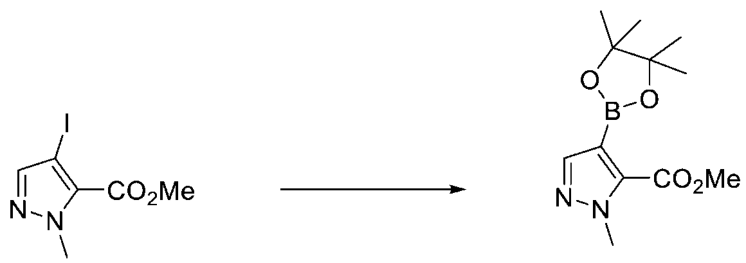

4-Iodo-1-methyl-1H-pyrazole-5-carboxylate (19.4 g) was dissolved in 1,4-dioxane (243 mL). Bis(pinacolato) diboron (27.8 g), [1,1'-bis(diphenylphosphino)ferrocene]palladium(II) dichloride dichloromethane adduct (3.0g) and potassium acetate (21.4 g) were added to the resultant solution, and then stirred overnight at 100°C under a nitrogen atmosphere. Water was added to the reaction solution, and filtered through Celite. The filtrate was extracted with ethyl acetate, and dried over anhydrous magnesium sulfate. The solvent was distilled off under reduced pressure, and the obtained residue was purified by silica gel column chromatography (developing solvent: n-hexane / ethyl acetate) to obtain 9.41 g of methyl 1-methyl-4-(4,4,5,5-tetramethyl-1,3,2-dioxaborolan-2-yl)-1H-pyrazole-5-carboxylate (at a yield of 49%).

Step 2: Conversion to methyl 4-hydroxy-1-methyl-1H-pyrazole-5-carboxylate (English name: methyl-4-hydroxy-1-methyl-1H-pyrazole-5-carboxylate)

[Chemical Formula 53]

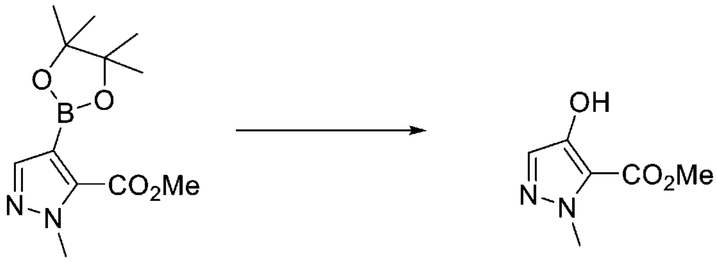

Methyl 1-methyl-4-(4,4,5,5-tetramethyl-1,3,2-dioxaborolan-2-yl)-1H-pyrazole-5-carboxylate (8.03 g) was dissolved in methanol (60 mL). Sodium carbonate (6.4 g) and 30% hydrogen peroxide (6.8 g) were added thereto, followed by stirring the mixture at room temperature for one hour.

A saturated aqueous solution of sodium thiosulfate and a saturated aqueous solution of ammonium chloride were added to the reaction liquid, and the mixture was extracted with a solution of dichloromethane / methanol = 9/1, and dried over anhydrous magnesium sulfate. The solvent was distilled off under reduced pressure, and the obtained residue was purified by silica gel column chromatography (developing solvent: n-hexane / ethyl acetate) to obtain 3.42 g of methyl 4-hydroxy-1-methyl-1H-pyrazole-5-carboxylate (at a yield of 73%).

Step 3: Conversion to methyl 4-(3-ethylphenoxy)-1-methyl-1H-pyrazole-5-carboxylate (English name: methyl 4-(3-ethylphenoxy)-1-methyl-1H-pyrazole-5-carboxylate)

[Chemical Formula 54]

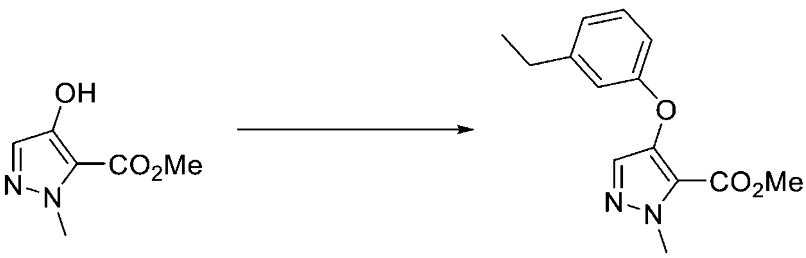

Methyl 4-hydroxy-1-methyl-1H-pyrazole-5-carboxylate (0.312 g) was dissolved in dichloromethane (10 mL). 3-Ethylphenylboronic acid (0.6 g), copper(II) acetate (0.6 g) and pyridine (0.24 g) were added thereto, followed by stirring the mixture at room temperature for two days.

The reaction liquid was distilled off under reduced pressure, and the obtained residue was purified by silica gel column chromatography (developing solvent: n-hexane / ethyl acetate) to obtain 0.46 g of methyl 4-(3-ethylphenoxy)-1-methyl-1H-pyrazole-5-carboxylate (at a yield of 88%).

Step 4: Conversion to 4-(3-ethylphenoxy)-1-methyl-1H-pyrazole-5-carboxylic acid (English name: 4-(3-ethylphenoxy)-1-methyl-1H-pyrazole-5-carboxylic acid)

[Chemical Formula 55]

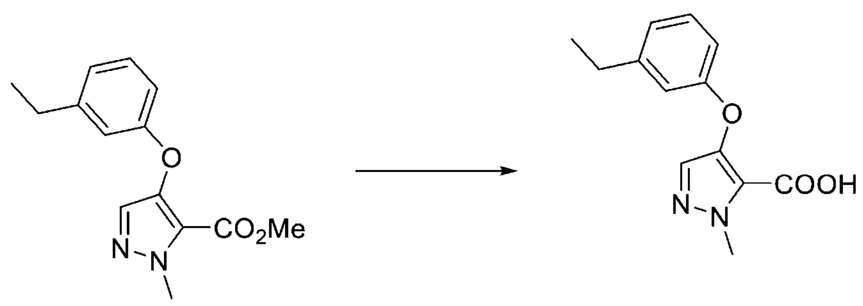

Methyl 4-(3-ethylphenoxy)-1-methyl-1H-pyrazole-5-carboxylate (0.46 g) was dissolved in a solution of tetrahydrofuran / methanol /water = 3/1/1 (9 mL). Lithium hydroxide monohydrate (0.31 g) was added thereto, and then stirred at room temperature for one hour.

An aqueous solution containing 1M hydrochloric acid was added to the reaction solution to adjust the pH thereof to 1. The acidic aqueous solution was extracted with ethyl acetate, and dried over anhydrous magnesium sulfate. The solvent was distilled off under reduced pressure to obtain 0.289 g of 4-(3-ethylphenoxy)-1-methyl-1H-pyrazole-5-carboxylic acid (at a yield of 66%).

Step 5: Conversion to N-(1-chloro-3-(2,4-dimethylphenyl)propan-2-yl)-4-(3-ethylphenoxy)-1-methyl-1H-pyrazole-5-carboxamide (English name: N-(1-chloro-3-(2,4-dimethylphenyl)propan-2-yl)-4-(3-ethylphenoxy)-1-methyl-1H-pyrazole-5-carboxamide

[Chemical Formula 56]

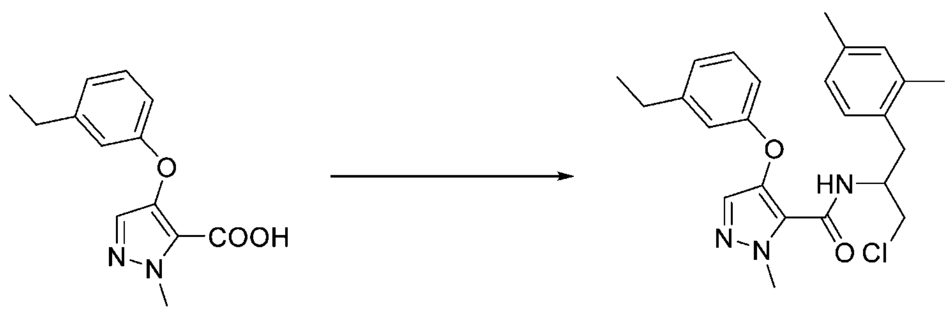

4-(3-Ethylphenoxy)-1-methyl-1H-pyrazole-5-carboxylic acid (0.13 g) was added to dichloromethane (5 mL). 1-Chloro-3-(2,4-dimethylphenyl)propan-2-amine hydrochloride (0.2 g), 1-(3-dimethylaminopropyl)-3-ethylcarbodiimide hydrochloride (0.17 g), 1-hydroxybenzotriazole (0.008 g) and triethylamine (0.12 g) were added thereto, and then stirred at room temperature for two hours.

A saturated ammonium chloride was added to the reaction liquid, and an organic layer was separated, and distilled off under reduced pressure. The obtained residue was purified by silica gel column chromatography (developing solvent: n-hexane / ethyl acetate) to obtain 0.2 g of N-(1-chloro-3-(2,4-dimethylphenyl)propan-2-yl)-4-(3-ethylphenoxy)-1-methyl-1H-pyrazole-5-carboxamide (at a yield of 81%).

Step 6: Conversion to N-(1-chloro-3-(2,4-dimethylphenyl)propan-2-yl)-4-(3-ethylphenoxy)-N'-hydroxy-1-methyl-1H-pyrazole-5-carboximidamide (English name: N-(1-chloro-3-(2,4-dimethylphenyl)propan-2-yl)-4-(3-ethylphenoxy)-N'-hydroxy-1-methyl-1H-pyrazole-5-carboximidamide)

[Chemical Formula 57]

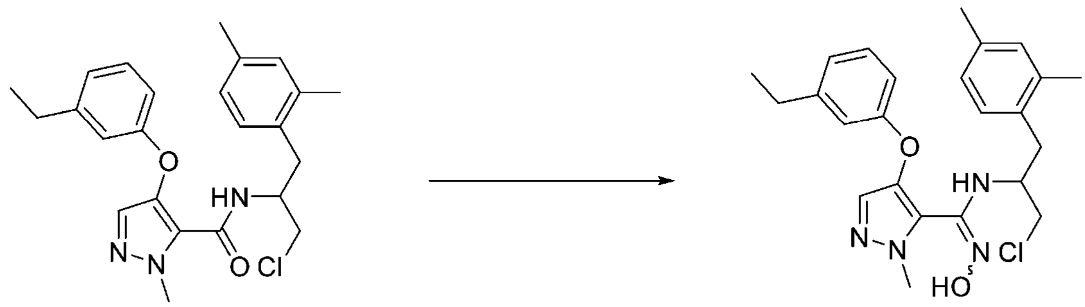

N-(1-Chloro-3-(2,4-dimethylphenyl)propan-2-yl)-4-(3-ethylphenoxy)-1-methyl-1H-pyrazole-5-carboxamide (0.2 g) was dissolved in toluene (5mL). Phosphorus pentachloride (0.39g) was added thereto, and stirred at 80°C for two hours under a nitrogen atmosphere. After the reaction liquid was distilled off under reduced pressure, toluene (5 mL) was added thereto and distilled off azeotropically. Acetonitrile (5 mL) was added to the resultant and stirred at 0°C, followed by adding hydroxylamine hydrochloride (0.31 g) and N,N-diisopropylethylamine (1.2 g) thereto, and then stirring the mixture at room temperature for 15 minutes.

The reaction liquid was acidified with an aqueous solution containing 1M hydrochloric acid, and then extracted with a solution of chloroform / methanol = 9/1. After the resultant was dried over anhydrous magnesium sulfate, the solvent was distilled off under reduced pressure. The obtained residue was purified by silica gel column chromatography (developing solvent: chloroform / methanol) to obtain 0.207 g of N-(1-chloro-3-(2,4-dimethylphenyl)propan-2-yl)-4-(3-ethylphenoxy)-N'-hydroxy-1-methyl-1H-pyrazole-5-carboximidamide (at a yield of 100%).

Step 7: Conversion to 5-(2,4-dimethylbenzyl)-3-(4-(3-ethylphenoxy)-1-methyl-1H-pyrazol-5-yl)-5,6-dihydro-4H-1,2,4-oxadiazine (English name: 5-(2,4-dimethylbenzyl)-3-(4-(3-ethylphenoxy)-1-methyl-1H-pyrazol-5-yl)-5,6-dihydro-4H-1,2,4-oxadiazine) (Compound No. a-10)

[Chemical Formula 58]

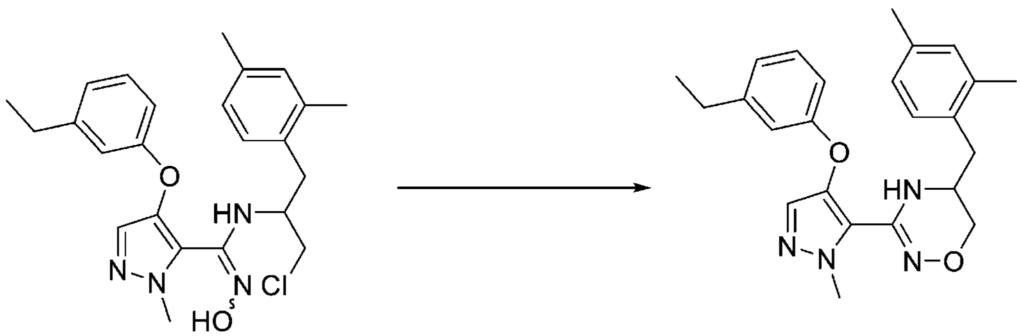

N-(1-chloro-3-(2,4-dimethylphenyl)propan-2-yl)-4-(3-ethylphenoxy)-N'-hydroxy-1-methyl-1H-pyrazole-5-carboximidamide (0.207 g) was dissolved in acetonitrile (3 mL), and then cesium carbonate (0.46 g) was added thereto, followed by stirring the mixture at room temperature overnight.

Water was added to the reaction liquid, the resultant was extracted with ethyl acetate and dried over anhydrous magnesium sulfate, and then the solvent was distilled off under reduced pressure. The obtained residue was purified by silica gel column chromatography (developing solvent: n-hexane / ethyl acetate) to obtain 0.062 g of 5-(2,4-dimethylbenzyl)-3-(4-(3-ethylphenoxy)-1-methyl-1H-pyrazol-5-yl)-5,6-dihydro-4H-1,2,4-oxadiazine (Compound No. a-10) (at a yield of 33%).

(Compound preparation example 6)

5-(2,4-Dimethylbenzyl)-3-(1-methyl-4-(3-(trifluoromethyl)phenoxy)-1H-1,2,3-triazol-5-yl)-5,6-dihydro-4H-1,2,4-oxadiazine (English name: 5-(2,4-dimethylbenzyl)-3-(1-methyl-4-(3-(trifluoromethyl)phenoxy)-1H-1,2,3-triazol-5-yl)-5,6-dihydro-4H-1,2,4-oxadiazine) (Compound No. a-14) was produced by the following steps.

Step 1: Conversion to isopropyl 1-methyl-4-(3-(trifluoromethyl)phenoxy)-1H-1,2,3-triazole-5-carboxylate (English name: isopropyl 1-methyl-4-(3-(trifluoromethyl)phenoxy)-1H-1,2,3-triazole-5-carboxylate)

[Chemical Formula 59]

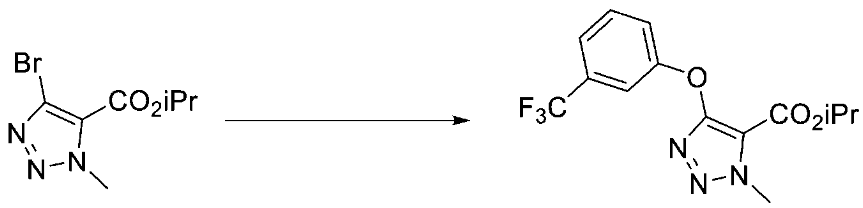

Isopropyl 4-bromo-1-methyl-1H-1,2,3-triazole-5-carboxylate (0.25 g) was dissolved in toluene (4.5mL). Copper iodide (19 mg), cesium carbonate (0.65 g), N,N-dimethylglycine (21 mg), 3-(trifluoromethyl)phenol (0.49g) were added thereto. The mixture was stirred for one hour at 165°C while irradiating microwave thereto.

The reaction liquid was directly purified by silica gel column chromatography (developing solvent: n-hexane / ethyl acetate) to obtain 0.14 g of isopropyl 1-methyl-4-(3-(trifluoromethyl)phenoxy)-1H-1,2,3-triazole-5-carboxylate (at a yield of 43%).

Step 2: Conversion to 1-methyl-4-(3-(trifluoromethyl)phenoxy)-1H-1,2,3-triazole5-carboxylic acid (English name: 1-methyl-4-(3-(trifluoromethyl)phenoxy)-1H-1,2,3-triazole-5-carboxylic acid)

[Chemical Formula 60]

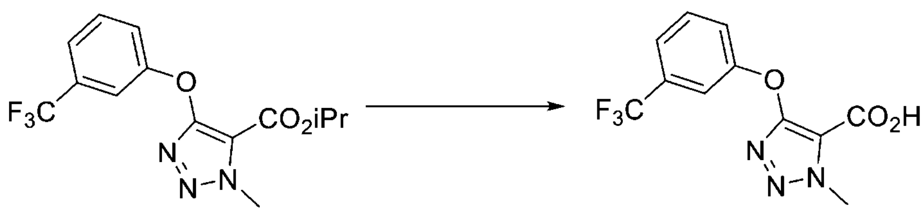

Isopropyl 1-methyl-4-(3-(trifluoromethyl)phenoxy)-1H-1,2,3-triazole-5-carboxylate (0.14 g) was dissolved in tetrahydrofuran (3 mL) and water (1 mL). Lithium hydroxide monohydrate (0.09 g) was added thereto, followed by stirring the mixture at room temperature for 18 hours.

An aqueous solution containing 1M hydrochloric acid was added to the reaction liquid, followed by extracting with ethyl acetate, and drying over anhydrous magnesium sulfate. The solvent was distilled off under reduced pressure to obtain 0.12 g of 1-methyl-4-(3-(trifluoromethyl)phenoxy)-1H-1,2,3-triazole-5-carboxylic acid (at a yield of 97%).

Step 3: Conversion to N-(1-chloro-3-(2,4-dimethylphenyl)propan-2-yl)-1-methyl-4-(3-(trifluoromethyl)phenoxy)-1H-1,2,3-triazole-5-carboxamide (English name: N-(1-chloro-3-(2,4-dimethylphenyl)propan-2-yl)-1-methyl-4-(3-(trifluoromethyl)phenoxy)-1H-1,2,3-triazole-5-carboxamide)

[Chemical Formula 61]

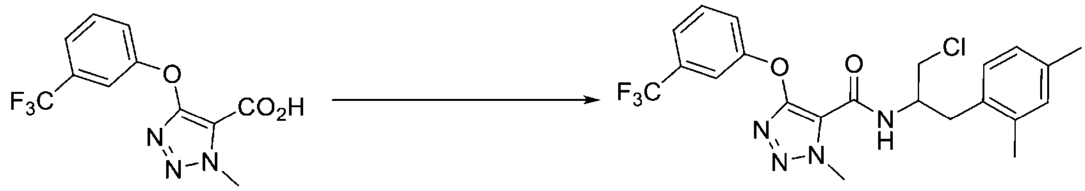

1-Methyl-4-(3-(trifluoromethyl)phenoxy)-1H-1,2,3-triazole-5-carboxylic acid (0.10 g) was added to dichloromethane (3 mL). 1-(3-Dimethylaminopropyl)-3-ethylcarbodiimide hydrochloride (90 mg), 1-chloro-3-(2,4-dimethylphenyl)propan-2-amine hydrochloride (0.10 g) and triethylamine (0.071 g) were added thereto, followed by stirring the mixture at room temperature for 17 hours.
The reaction solvent was distilled off under reduced pressure, and then purified by silica gel column chromatography (developing solvent: n-hexane /ethyl acetate) to obtain 0.08 g of N-(1-chloro-3-(2,4-dimethylphenyl)propan-2-yl)-1-methyl-4-(3-(trifluoromethyl)phenoxy)-1H-1,2,3-triazole-5-carboxamide (at a yield of 49%).

Step 4: Conversion to N-(1-chloro-3-(2,4-dimethylphenyl)propan-2-yl)-N'-hydroxy-1-methyl-4-(3-(trifluoromethyl)phenoxy)-1H-1,2,3-triazole-5-carboximidamide (English name: N-(1-chloro-3-(2,4-dimethylphenyl)propan-2-yl)-N'-hydroxy-1-methyl-4-(3-(trifluoromethyl)phenoxy)-1H-1,2,3-triazole-5-carboximidamide)

[Chemical Formula 62]

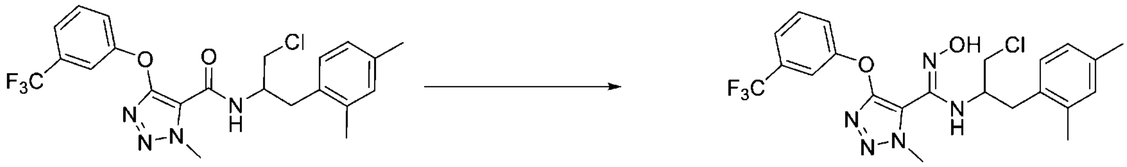

N-(1-chloro-3-(2,4-dimethylphenyl)propan-2-yl)-1-methyl-4-(3-(trifluoromethyl)phenoxy)-1H-1,2,3-triazole-5-carboxamide (0.09 g) was dissolved in toluene (2mL). Phosphorus pentachloride (0.16 g) was added thereto, followed by stirring the mixture at 80°C for two hours under a nitrogen atmosphere.
After the reaction liquid was distilled off under reduced pressure, acetonitrile (3 mL) was added to the resultant, and stirred at 0°C, followed by adding hydroxylamine hydrochloride (0.26 g) and N,N-diisopropylethylamine (0.49 g) to the resultant, and then stirring the mixture at room temperature for 15 minutes.
After the reaction solvent was distilled off under reduced pressure, the resultant was purified by silica gel column chromatography (developing solvent: chloroform / methanol) to obtain 0.04 g of N-(1-chloro-3-(2,4-dimethylphenyl)propan-2-yl)-N'-hydroxy-1-methyl-4-(3-(trifluoromethyl)phenoxy)-1H-1,2,3-triazole-5-carboximidamide (at a yield of 44%).

Step 5: Conversion to 5-(2,4-dimethylbenzyl)-3-(1-methyl-4-(3-(trifluoromethyl)phenoxy)-1H-1,2,3-triazol-5-yl)-5,6-dihydro-4H-1,2,4-oxadiazine (English name: 5-(2,4-dimethylbenzyl)-3-(1-methyl-4-(3-(trifluoromethyl)phenoxy)-1H-1,2,3-triazol-5-yl)-5,6-dihydro-4H-1,2,4-oxadiazine) ( Compound No. a-14)

[Chemical Formula 63]

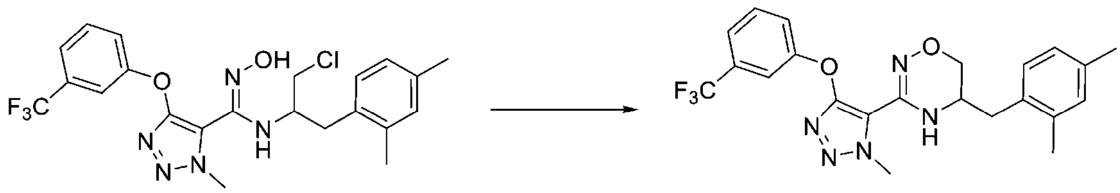

N-(1-chloro-3-(2,4-dimethylphenyl)propan-2-yl)-N'-hydroxy-1-methyl-4-(3-(trifluoromethyl)phenoxy)-1H-1,2,3-triazole-5-carboximidamide (0.04 g) was dissolved in acetonitrile(3 mL), and then cesium carbonate (0.08 g) was added thereto, followed by stirring the mixture at room temperature overnight.

After the reaction solvent was distilled off under reduced pressure, the resultant was purified by silica gel column chromatography (developing solvent: n-hexane / ethyl acetate) to obtain 0.037 g of 5-(2,4-dimethylbenzyl)-3-(1-methyl-4-(3-(trifluoromethyl)phenoxy)-1H-1,2,3-triazol-5-yl)-5,6-dihydro-4H-1,2,4-oxadiazine (Compound No. a-14) (at a yield of 100%).

Compounds shown in the following Table 1 were produced through the same steps as those in the above-mentioned examples.

The LCMS retention time (SQD2) was determined by the following method.

The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (SQ Detector 2 detector (positive and negative ion electrospray modes and an atmospheric pressure ionization mode), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

The LCMS (QDa) retention time was determined by the following method.

The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (ACQUITY QDa detector (positive and negative ion electrospray modes), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

[Table 1]

Table 1

| Compound No. | Formula | External appearance | LCMS Retention time | LCMS Condition |
|---|---|---|---|---|
| a-1 | 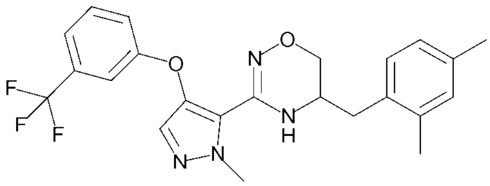 | VISCOUS OIL | 1.83 min | SQD2 |
| a-2 | 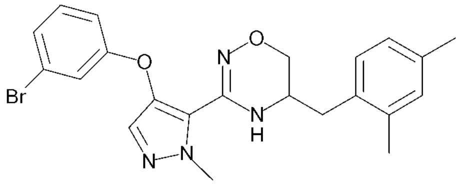 | VISCOUS OIL | 1.86 min | SQD2 |
| a-3 | 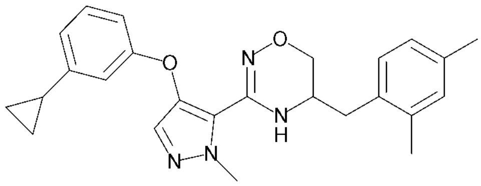 | VISCOUS OIL | 1.89 min | SQD2 |
| a-4 | 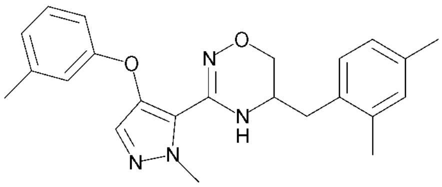 | VISCOUS OIL | 1.82 min | SQD2 |

[Table 2]

Table 1 (continued)

| Compound No. | Formula | External appearance | LCMS Retention time | LCMS Condition |
|---|---|---|---|---|
| a-5 | 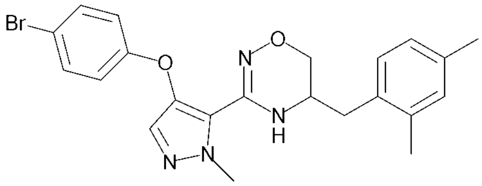 | VISCOUS OIL | 1.83 min | SQD2 |
| a-6 | 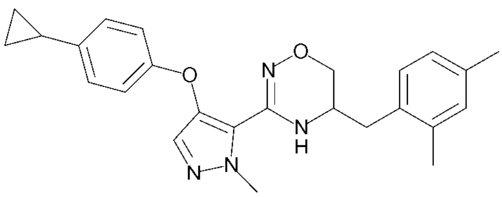 | VISCOUS OIL | 1.96 min | SQD2 |

(continued)

Table 1 (continued)

| Compound No. | Formula | External appearance | LCMS Retention time | LCMS Condition |
|---|---|---|---|---|
| a-7 | 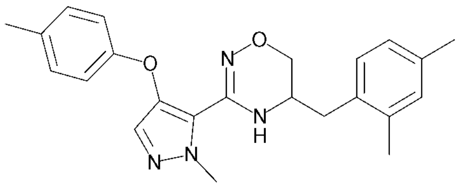 | VISCOUS OIL | 1.88 min | SQD2 |
| a-8 | 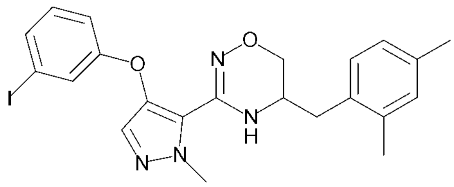 | VISCOUS OIL | 1.92 min | SQD2 |

[Table 3]

Table 1 (continued)

| Compound No. | Formula | External appearance | LCMS Retention time | LCMS Condition |
|---|---|---|---|---|
| a-9 | 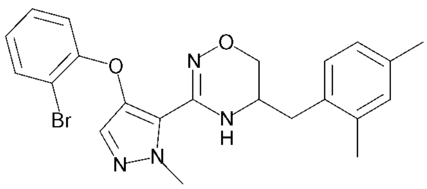 | VISCOUS OIL | 1.88 min | SQD2 |
| a-10 | 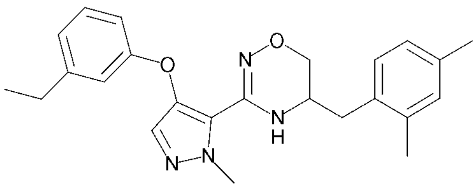 | VISCOUS OIL | 1.88 min | Qda |
| a-11 | 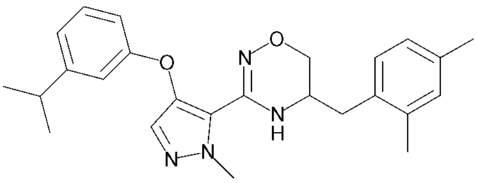 | VISCOUS OIL | 1.86 min | Qda |
| a-12 | 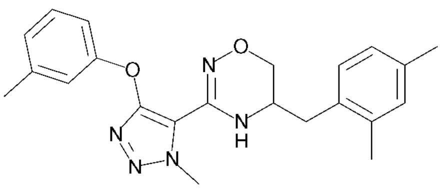 | VISCOUS OIL | 1.75 min | SQD2 |

[Table 4]

Table 1 (continued)

| Compound No. | Formula | External appearance | LCMS Retention time | LCMS Condition |
|---|---|---|---|---|
| a-13 | 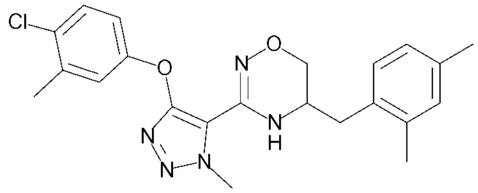 | VISCOUS OIL | 1.87 min | SQD2 |
| a-14 | 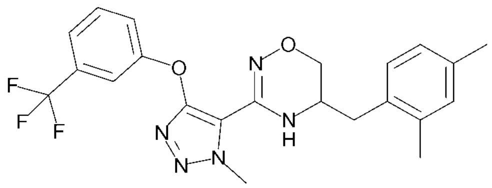 | VISCOUS OIL | 1.79 min | SQD2 |
| a-15 | 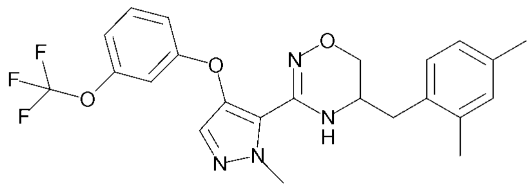 | VISCOUS OIL | 1.86 min | SQD2 |
| a-16 | 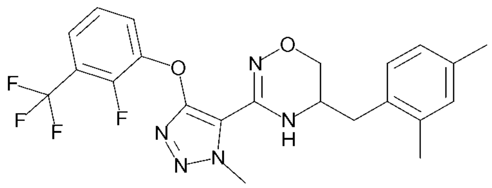 | VISCOUS OIL | 1.80 min | SQD2 |

[Table 5]

Table 1 (continued)

| Compound No. | Formula | External appearance | LCMS Retention time | LCMS Condition |
|---|---|---|---|---|
| a-17 | 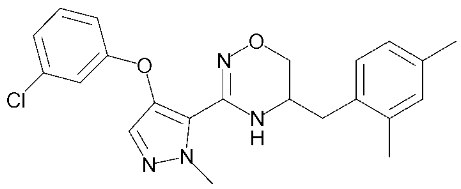 | VISCOUS OIL | 1.80 min | SQD2 |
| a-18 | 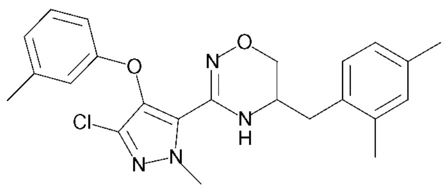 | VISCOUS OIL | 1.93 min | SQD2 |

(continued)

Table 1 (continued)

| Compound No. | Formula | External appearance | LCMS Retention time | LCMS Condition |
|---|---|---|---|---|
| a-19 | 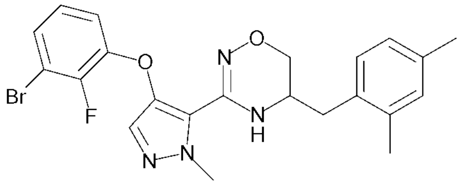 | VISCOUS OIL | 1.83 min | SQD2 |
| a-20 | 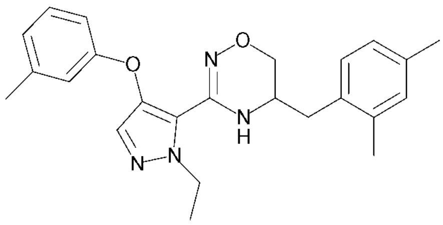 | VISCOUS OIL | 1.88 min | SQD2 |

[Table 6]

Table 1 (continued)

| Compound No. | Formula | External appearance | LCMS Retention time | LCMS Condition |
|---|---|---|---|---|
| a-21 | 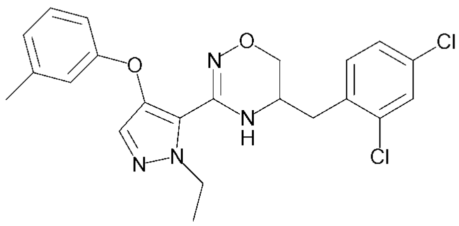 | VISCOUS OIL | 1.91 min | SQD2 |
| a-22 | 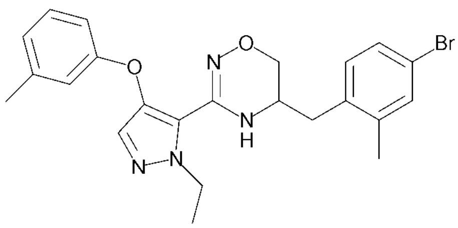 | VISCOUS OIL | 1.91 min | SQD2 |
| a-23 | 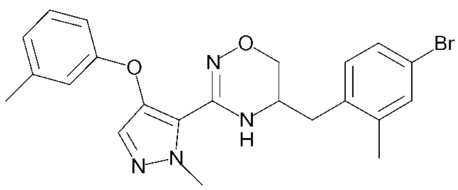 | VISCOUS OIL | 1.84 min | SQD2 |

(continued)

Table 1 (continued)

| Compound No. | Formula | External appearance | LCMS Retention time | LCMS Condition |
|---|---|---|---|---|
| a-24 | 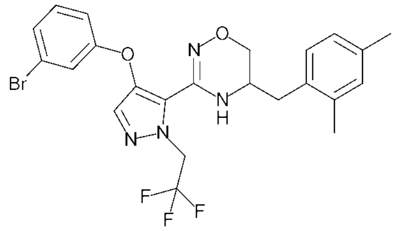 | VISCOUS OIL | 2.03 min | SQD2 |

[Table 7]

Table 1 (continued)

| Compound No. | Formula | External appearance | LCMS Retention time | LCMS Condition |
|---|---|---|---|---|
| a-25 | 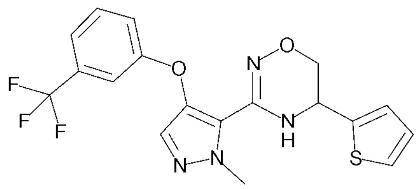 | VISCOUS OIL | 1.62 min | SQD2 |
| a-26 | 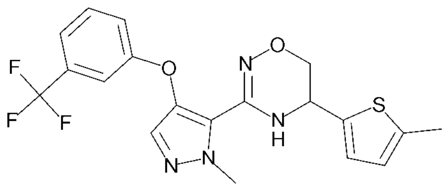 | AMORPHOUS | 1.67 min | SQD2 |
| a-27 | 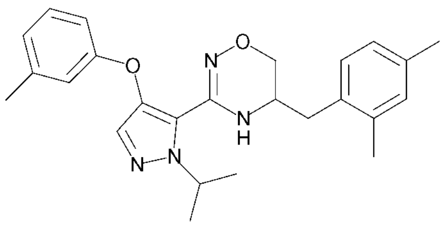 | VISCOUS OIL | 1.95 min | SQD2 |
| a-28 | 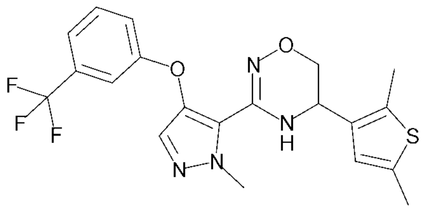 | AMORPHOUS | 1.74 min | SQD2 |

[Table 8]

Table 1 (continued)

| Compound No. | Formula | External appearance | LCMS Retention time | LCMS Condition |
|---|---|---|---|---|
| a-29 | 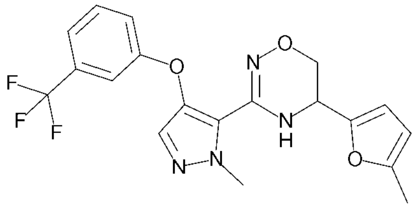 | AMORPHOUS | 1.58 min | SQD2 |
| a-30 | 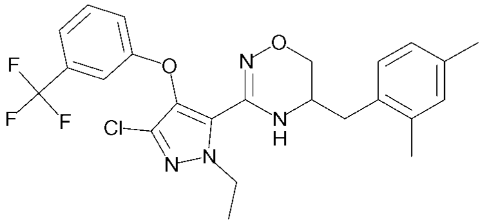 | AMORPHOUS | 2.09 min | SQD2 |
| a-31 | 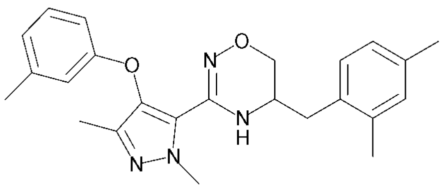 | VISCOUS OIL | 1.87 min | SQD2 |
| a-32 | 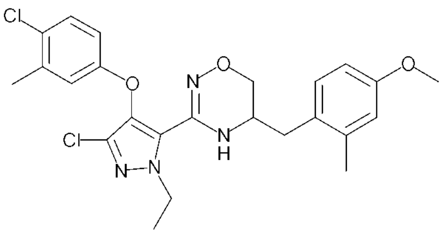 | AMORPHOUS | 1.96 min | SQD2 |

[Table 9]

Table 1 (continued)

| Compound No. | Formula | External appearance | LCMS Retention time | LCMS Condition |
|---|---|---|---|---|
| a-33 | 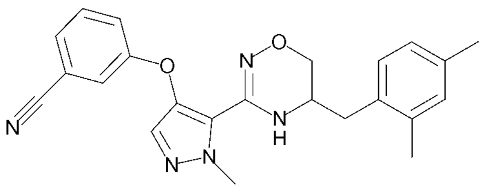 | VISCOUS OIL | *1.55* min | SQD2 |
| a-34 | 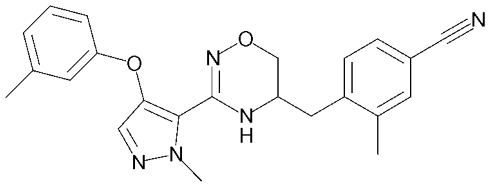 | VISCOUS OIL | 1.54 min | SQD2 |

(continued)

Table 1 (continued)

| Compound No. | Formula | External appearance | LCMS Retention time | LCMS Condition |
|---|---|---|---|---|
| a-35 | 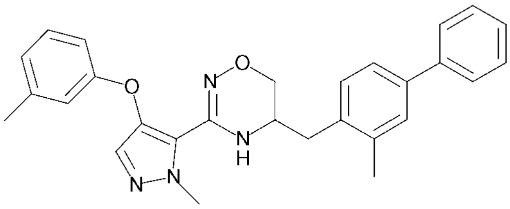 | VISCOUS OIL | 1.97 min | SQD2 |
| a-36 | 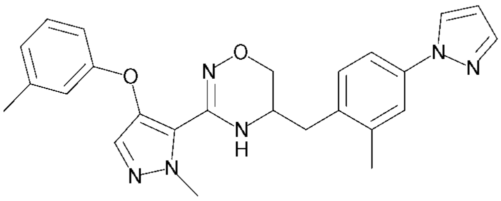 | VISCOUS OIL | 1.60 min | SQD2 |

Effects of the present invention are exemplified by the following fungicidal effect test and disease control test.

(Fungicidal effect test against fungus causing gray mold)

A spore suspension of fungus causing gray mold disease (Botrytis cinerea, Vogel medium) was prepared. A five-membered heteroaryl compound according to the present invention was added to dimethyl sulfoxide to obtain a drug liquid. This drug liquid and the spore suspension were mixed in equal amounts (based on volume) to obtain a test liquid in which the concentration of the five-membered heteroaryl compound according to the present invention was 25 ppm by volume. The test liquid was added dropwise into a 96-well microplate (area treated with drug).
Dimethyl sulfoxide and the spore suspension were mixed in equal amounts (based on volume) to obtain a control liquid. The control liquid was added dropwise into a 96-well microplate (untreated area).
The microplate was left still in a dark place at 20°C for five days to carry out cultivation. The turbidity OD at a wavelength of 405 nm was measured using a microplate reader. The growth inhibition ratio was calculated by the following equation.

Growth inhibition ratio (%) = 100-100× {[(OD of area treated with drug after cultivation)-(OD of area treated with drug before cultivation)] / [(OD of untreated area after cultivation)-(OD of untreated area before cultivation)]}

Compound Nos. a-1 to a-4, a-6 to a-10, a-12, a-14 to a-24, a-26 to a-28, and a-30 to a-36 were subjected to the fungicidal effect test against fungus causing gray mold. The growth inhibition ratios of all compounds were at least 75%.

(Fungicidal effect test against fungus causing apple anthracnose)

The turbidity was measured by the same method as the fungicidal effect test against fungus causing gray mold, except that the spore suspension of fungus causing gray mold disease (Botrytis cinerea, Vogel medium) was replaced with a spore suspension of fungus causing apple anthracnose (Colletotrichum acutatum, Czapek medium), and the temperature and the time period when left still were changed to 25°C and four days, to calculate the growth inhibition ratio.

Compound Nos. a-1 to a-5, a-7, a-8, a-10 to a-14, a-16 to a-24, a-26 to a-28, and a-30 to a-36 were subjected to a fungicidal effect test against fungi causing apple anthracnose. The growth inhibition ratios of all compounds were at least 75%.

(Fungicidal effect test against fungi causing grape ripe rot disease)

The turbidity was measured by the same method as the fungicidal effect test against fungus causing gray mold, except that the spore suspension of fungus causing gray mold disease (Botrytis cinerea, Vogel medium) was replaced with a spore suspension of fungus causing grape ripe rot disease (Glomerella cingulata, Vogel medium), and the temperature and the time period when left still were changed to 25°C and three days, to calculate the growth inhibition ratio.
Compound Nos. a-1 to a-8, a-10 to a-14, and a-16 to a-36 were subjected to the fungicidal effect test against fungi causing grape ripe rot disease. The growth inhibition ratios of all compounds were at least 75%.

(Fungicidal effect test against fungi causing wheat Fusarium head blight)

The turbidity was measured by the same method as the fungicidal effect test against fungus causing gray mold, except that the spore suspension of fungus causing gray mold disease (Botrytis cinerea, Vogel medium) was replaced with a spore suspension of fungi causing wheat Fusarium head blight disease (Fusarium graminearum, SD medium), and the temperature and the time period when left still were changed to 25°C and four days, to calculate the growth inhibition ratio.
Compound Nos. a-1, a-2, a-4, a-12, a-14, a-17 to a-24, a-27, a-30, a-31, and a-33 were subjected to the fungicidal effect test against fungi causing wheat Fusarium head blight. The growth inhibition ratios of all compounds were at least 75%.

(Control test of cucumber gray mold)

5 parts by mass of a five-membered heteroaryl compound according to the present invention, 93.5 parts by mass of N,N-dimethylformamide, and 1.5 parts by mass of polyoxyethylene sorbitan monolaurate were mixed together to obtain an emulsion having 5% by mass of the active ingredient.
Water was added to the emulsion to obtain a drug liquid in which the concentration of the five-membered heteroaryl compound according to the present invention reached a predetermined concentration. Then, the drug liquid was sprayed onto young cucumber seedlings cultivated in a nursery pot (variety "Jihai"-based, cotyledon stage), and then air-dried. Then, a conidiospore suspension of fungus causing cucumber gray mold disease (Botrytis cinerea) was inoculated dropwise thereon (treated area). As a control, a conidiospore suspension of fungus causing cucumber gray mold disease (Botrytis cinerea) was inoculated dropwise onto young cucumber seedlings unsprayed with the drug liquid (untreated area). They were left still in a moist room at 20°C. Four days after inoculation, leaves of the young cucumber seedlings were visually observed, and the control value was calculated from the lesion area ratio in accordance with the following equation.

$$\text{Control value (\%)} = 100-\{\text{the lesion area ratio at the treated area / the lesion area ratio at the untreated area}\} \times 100$$

Compound Nos. a-1, a-3, a-4, a-8, a-12, a-14, a-18 to a-23, a-27, a-30, and a-34 were subjected to the control test of cucumber gray mold at the concentration of 125 ppm by mass. The control values of all compounds were at least 75%.
Compound Nos. a-1, a-2, a-4, a-12, a-14, a-17 to a-24, a-27, and a-30 were subjected to the control test of cucumber gray mold at the concentration of 25 ppm by mass. The control values of all compounds were at least 75%.

(Prevention test of cucumber sclerotinia rot disease)

Water was added to the emulsion such that the concentration of the five-membered heteroaryl compound according to the present invention became 100 ppm by mass, and the five-membered heteroaryl compound was dissolved to obtain a drug liquid. Thereafter, the drug liquid was sprayed onto young cucumber seedlings grown in nursery pots (variety "Jihai"-based, cotyledon stage), and then air-dried. Thereafter, the young cucumber seedlings were inoculated with a mycelia of fungi causing sclerotinia rot disease (Sclerotinia sclerotiorum) grown on an agar medium (treated area). As a control, young cucumber seedlings unsprayed with the drug liquid were inoculated with a mycelia of fungi causing

sclerotinia rot disease (Sclerotinia sclerotiorum) grown on an agar medium (untreated area).

They were left still at 20°C in a moist room. Four days after inoculation, leaves of the young cucumber seedlings were visually observed, and the control value was calculated from the lesion area ratio in accordance with the equation used in the control test of cucumber gray mold.

Compound Nos. a-1, a-4, a-8, a-14, and a-18 to a-24 were subjected to the prevention test of cucumber sclerotinia rot disease. The growth inhibition ratios of all compounds were at least 75%.

(Residual effect test against cucumber gray mold)

Water was added to the emulsion such that the concentration of the five-membered heteroaryl compound according to the present invention became 400 ppm by mass, and the five-membered heteroaryl compound was dissolved to obtain a drug liquid. Then, the drug liquid was sprayed onto young cucumber seedlings grown in nursery pots (variety "Jihai"-based, cotyledon stage), and then air-dried, followed by leaving them still in a growth chamber for seven day. Thereafter, a conidiospore suspension of fungus causing cucumber gray mold disease (Botrytis cinerea) was inoculated dropwise thereon (treated area). As a control, a conidiospore suspension of fungus causing cucumber gray mold disease (Botrytis cinerea) was inoculated dropwise onto young cucumber seedlings unsprayed with the drug liquid (untreated area).

They were left still at 20°C in a moist room. Four days after inoculation, leaves of the young cucumber seedlings were visually observed, and the control value was calculated from the lesion area ratio in accordance with the equation used in the control test of cucumber gray mold.

Compound Nos. a-18, a-21, a-22, and a-27 were subjected to the residual effect test against cucumber gray mold. The growth inhibition ratios of all compounds were at least 75%.

Since all compounds selected from the five-membered heteroaryl compounds according to the present invention at random exhibited the above-mentioned effects, it can be understood that the five-membered heteroaryl compounds according to the present invention, encompassing compounds not listed as examples, have fungicidal effects, do not cause phytotoxicity to plants, and exhibit low toxicity to humans, livestock, and fish, and have low impact on the environment.

INDUSTRIAL APPLICABILITY

The five-membered heteroaryl compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively control diseases of agricultural and horticultural plants.

## Claims

1. A compound of formula (I), a stereoisomer thereof, or a salt thereof,

[Chemical Formula 1]

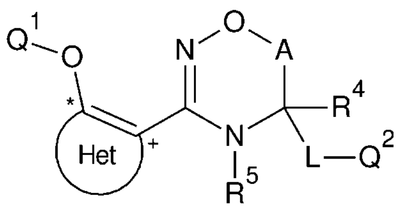

(I)

(in the formula (I),
a partial structure:

[Chemical Formula 2]

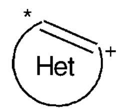

is a substituted or unsubstituted five-membered heteroaryl ring, carbon atoms indicated with * and + in the partial structure correspond to carbon atoms indicated with * and + in the formula (I),

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$R^4$ is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
$R^5$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group, and
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.)

**2.** The compound, the stereoisomer thereof, or the salt thereof, according to claim 1, wherein the partial structure:

[Chemical Formula 3]

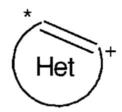

is of formula (II):

[Chemical Formula 4]

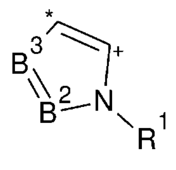

(II)

(in the formula (II),

$R^1$ is a substituted or unsubstituted C1-6 alkyl group,
$B^2$ is a nitrogen atom, or a group of formula: $CR^2$,
$R^2$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted

C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group,

$B^3$ is a nitrogen atom, or a group of formula: $CR^3$,

$R^3$ is a hydrogen atom, a substituted or unsubstituted C1-6 alkyl group, a substituted or unsubstituted C2-6 alkenyl group, a substituted or unsubstituted C2-6 alkynyl group, a hydroxy group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five- or six-membered heterocyclyl group, a substituted or unsubstituted phenyloxy group, a substituted or unsubstituted naphthyloxy group, a substituted or unsubstituted five- or six-membered heterocyclyloxy group, a substituted or unsubstituted amino group, a substituted or unsubstituted aminocarbonyl group, a nitro group, a cyano group, or a halogeno group, and

carbon atoms indicated with * and + correspond to carbon atoms indicated with * and + in the formula (I)).

**3.** An agricultural and horticultural fungicide comprising at least one selected from the group consisting of a compound, a stereoisomer thereof, and a salt thereof, of claim 1 or 2, as an active ingredient.

**4.** A method for producing a compound of formula (I-1), comprising

reacting a compound of formula (4) with a compound of formula (5) or a salt thereof to obtain a compound of formula (3), treating the compound of formula (3) with a halogenating agent, and then with hydroxyamine or a salt thereof to obtain a compound of formula (2), and then cyclizing the compound of formula (2) in the presence of a base to produce the compound of formula (I-1),

[Chemical Formula 5]

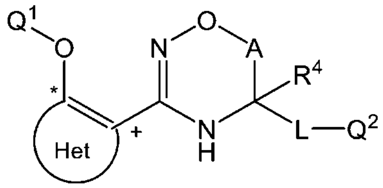

(I－1)

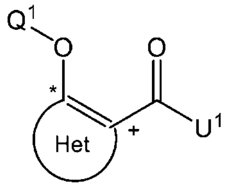

(4)

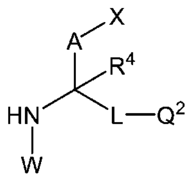

(5)

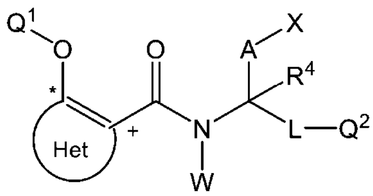

(3)

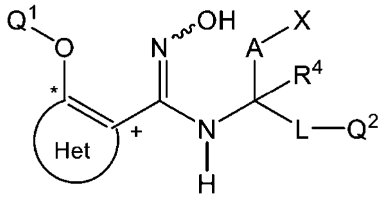

(2)

(in each formula,
a partial structure:

[Chemical Formula 6]

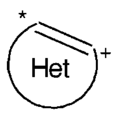

is a substituted or unsubstituted five-membered heteroaryl ring, carbon atoms indicated with * and + in the partial structure correspond to carbon atoms indicated with * and + in the formula (I),

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
$R^4$ is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
A is a substituted or unsubstituted methylene group, or a substituted or unsubstituted dimethylene group,
L is a single bond, a substituted or unsubstituted methylene group, or a substituted or unsubstituted dimethylene group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered

heterocyclyl group,
$U^1$ is a hydroxy group or halogeno group,
X is a halogeno group, and
W is a hydrogen atom, a t-butoxycarbonyl group, a benzyl group, an allyl group, or a (4-methoxyphenyl)methyl group.)

**5.** A method for producing a compound of formula (I-2), comprising
reacting a compound of formula (7) with a compound of formula (8) to obtain a compound of formula (6), and treating the compound of formula (6) with a reducing agent under acidic conditions to allow cyclization to proceed, thereby producing the compound of formula (I-2),

[Chemical Formula 7]

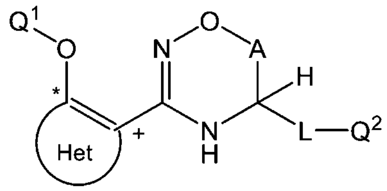

(I-2)

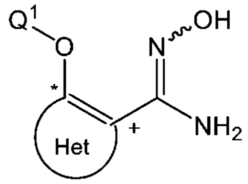

(7)

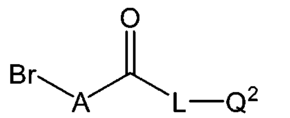

(8)

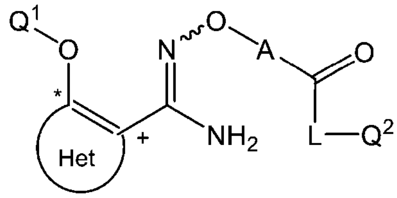

(6)

(in each formula,

a partial structure:

[Chemical Formula 8]

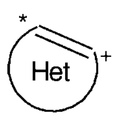

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group, and
$Q^2$ is a substituted or unsubstituted phenyl group, a substituted or unsubstituted naphthyl group, a substituted or

unsubstituted five- or six-membered heterocyclyl group, or a substituted or unsubstituted nine- or ten-membered heterocyclyl group.)

**6.** A method for producing a compound of formula (I-3), comprising reacting a compound of formula (7) with a compound of formula (12) to obtain a compound of formula (9), treating the compound of formula (9) under oxidizing conditions with osmium trioxide and sodium periodate to allow cyclization to proceed, thereby obtaining a compound of formula (10), followed by reacting the compound of formula (10) with a compound of formula (11) under acidic conditions to produce the compound of formula (I-3),

[Chemical Formula 9]

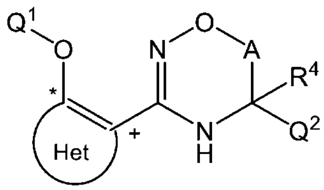

(I − 3)

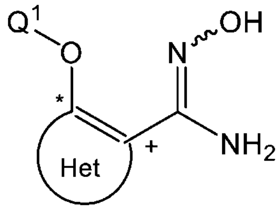

(7)

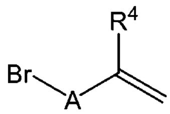

(1 2)

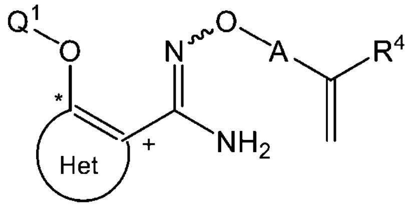

(9)

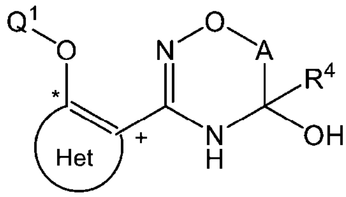

(1 0)

$H-Q^2$ (1 1)

(in each formula,

a partial structure:

[Chemical Formula 10]

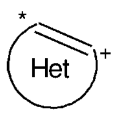

is a substituted or unsubstituted five-membered heteroaryl ring, carbon atoms indicated with * and + in the partial

structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group,
A is a substituted or unsubstituted methylene group, or a substituted or unsubstituted dimethylene group, and
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group.)

**7.** A method for producing a compound of formula (I), comprising
reacting a compound of formula (22) with a compound of formula (23) or a salt thereof to obtain a compound of formula (21), followed by allowing cyclization to proceed in the presence of a base, thereby producing the compound of formula (I),

[Chemical Formula 11]

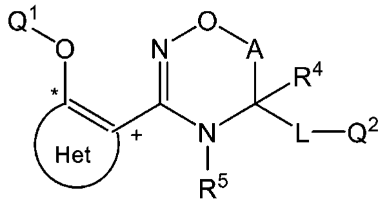

(I)

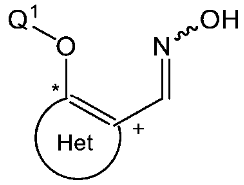

(22)

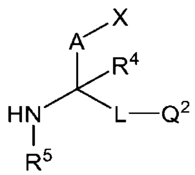

(23)

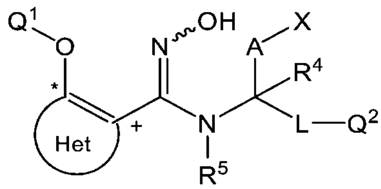

(21)

(in each formula,

a partial structure:

[Chemical Formula 12]

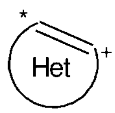

is a substituted or unsubstituted five-membered heteroaryl ring, carbon atoms indicated with * and + in the partial structure correspond to carbon atoms indicated with * and + in the formula (I),
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered

heterocyclyl group,
$R^4$ is a hydrogen atom, a substituted or unsubstituted C1-6 alkyl group, a hydroxy group, a substituted or unsubstituted C1-6 alkoxy group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, or a substituted or unsubstituted C1-6 alkylsulfonyl group,
$R^5$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five- or six-membered heterocyclyl group,
A is a substituted or unsubstituted methylene group, or a substituted or unsubstituted dimethylene group,
L is a single bond, a substituted or unsubstituted methylene group, or a substituted or unsubstituted dimethylene group,
$Q^2$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group, and
X is a halogeno group.)

**8.** A compound of formula (7):

[Chemical Formula 13]

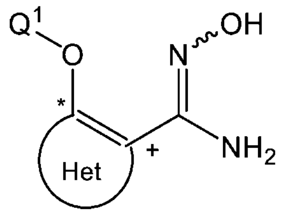

(7)

(in the formula (7),

a partial structure:

[Chemical Formula 14]

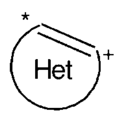

is a substituted or unsubstituted five-membered heteroaryl ring, carbon atoms indicated with * and + in the partial structure correspond to carbon atoms indicated with * and + in the formula (7), and
$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group).

**9.** A compound of formula (22):

[Chemical Formula 15]

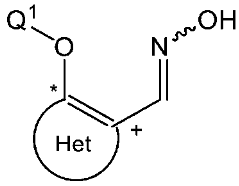

(2 2)

(in the formula (22),

a partial structure:

[Chemical Formula 16]

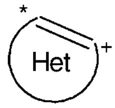

is a substituted or unsubstituted five-membered heteroaryl ring, carbon atoms indicated with * and + in the partial structure correspond to carbon atoms indicated with * and + in the formula (22), and

$Q^1$ is a substituted or unsubstituted phenyl group, a substituted or unsubstituted naphthyl group, a substituted or unsubstituted five- or six-membered heterocyclyl group, or a substituted or unsubstituted nine- or ten-membered heterocyclyl group).

## INTERNATIONAL SEARCH REPORT

International application No.
**PCT/JP2023/046591**

**A. CLASSIFICATION OF SUBJECT MATTER**

***C07D 413/04***(2006.01)i; ***A01N 43/88***(2006.01)i; ***A01P 3/00***(2006.01)i; ***C07D 231/18***(2006.01)i
FI: C07D413/04 CSP; A01P3/00; A01N43/88 102; C07D231/18

According to International Patent Classification (IPC) or to both national classification and IPC

**B. FIELDS SEARCHED**

Minimum documentation searched (classification system followed by classification symbols)

C07D413/04; A01N43/88; A01P3/00; C07D231/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CN 1188764 A (SHENYANG CHEMICAL INST., MINISTRY OF CHEMICAL INDUSTRY) 29 July 1998 (1998-07-29)<br>example 8, starting compound, intermediate | 8-9 |
| X | CAS Registry No. 1993745-56-4 et al., Database Registry [online], 15 September 2016, the issuance date is based on the latest compound of registration. [retrieved on 14 March 2024], retrieved from: STN<br>entire text | 8 |
| X | CN 111925359 A (NANTONG UNIVERSITY) 13 November 2020 (2020-11-13)<br>claim 2 | 9 |
| X | CN 111393413 A (NANTONG UNIVERSITY) 10 July 2020 (2020-07-10)<br>claim 2 | 9 |
| X | CN 104725366 A (NANTONG UNIVERSITY) 24 June 2015 (2015-06-24)<br>claim 2, formula II, examples | 9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2024** | **02 April 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No.
PCT/JP2023/046591

C. DOCUMENTS CONSIDERED TO BE RELEVANT

| Category* | Citation of document, with indication, where appropriate, of the relevant passages | Relevant to claim No. |
|---|---|---|
| X | JP 2015-86179 A (SUMITOMO CHEMICAL COMPANY, LIMITED) 07 May 2015 (2015-05-07) paragraph [0014], compound (2), reference production examples 1-3 | 9 |
| X | WO 2008/093639 A1 (TAKEDA PHARMACEUTICAL COMPANY LIMITED.) 07 August 2008 (2008-08-07) reference examples 194, 195, examples 103, 104, 131, 134, 169, 170, 176, 177, 191, 192, 213, 214, 216, 217, 226, 227 | 9 |
| X | DE 4200742 A1 (CIBA-GEIGY AG, BASEL, CH) 23 July 1992 (1992-07-23) page 10, table 2 | 9 |
| X | JP 2-96568 A (ISHIHARA SANGYO KAISHA, LTD.) 09 April 1990 (1990-04-09) page 10, tables 1, 2 | 9 |
| X | JP 1-197471 A (NISSAN CHEM. IND. LTD.) 09 August 1989 (1989-08-09) page 3, formula II, production example 1 | 9 |
| X | JP 63-183564 A (NIPPON NOHYAKU CO., LTD.) 28 July 1988 (1988-07-28) claim 2, examples | 9 |
| X | JP 62-53969 A (NIPPON NOHYAKU CO., LTD.) 09 March 1987 (1987-03-09) claim 1, table 1, examples 1-3 | 9 |
| X | DAI, Hong et al., Synthesis and Bioactivities of Novel Pyrazole Oxime Derivatives Containing a 5-Trifluoromethylpyridyl Moiety, Molecules, 2016, 21(3), 276/1-276/12, ISSN: 1420-3049, DOI:10.3390/molecules21030276 scheme 1, compounds 7a-7w, table 1 | 9 |
| A | WO 2021/245087 A1 (BAYER AKTIENGESELLSCHAFT) 09 December 2021 (2021-12-09) | 1-9 |
| A | US 3468884 A (ABBOTT LAB.) 23 September 1969 (1969-09-23) | 1-9 |
| A | US 3574201 A (E.R. SQUIBB & SONS INC.) 06 April 1971 (1971-04-06) | 1-9 |

Form PCT/ISA/210 (second sheet) (July 2022)

**INTERNATIONAL SEARCH REPORT**

International application No.
**PCT/JP2023/046591**

**Box No. III Observations where unity of invention is lacking (Continuation of item 3 of first sheet)**

This International Searching Authority found multiple inventions in this international application, as follows:

The claims are classified into the following three inventions.

(Invention 1) Claims 1-7

Independent claim 1 and claims 2 and 3 dependent thereon have the special technical feature of a compound represented by formula (I), independent claims 4-7 also share the same technical feature, and thus claims 1-7 are classified as invention 1.

(Invention 2) Claim 8

Independent claim 8 lacks novelty in light of documents 1 and 2 and thus does not have a special technical feature.

Claim 8 is therefore classified as invention 2.

(Invention 3) Claim 9

Independent claim 9 lacks novelty in light of documents 1 and 3-13, and thus does not have a special technical feature.

Claim 9 is therefore classified as invention 3.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2023/046591**

| Patent document cited in search report | Publication date (day/month/year) | Patent family member(s) | Publication date (day/month/year) |
|---|---|---|---|
| CN 1188764 A | 29 July 1998 | (Family: none) | |
| CN 111925359 A | 13 November 2020 | (Family: none) | |
| CN 111393413 A | 10 July 2020 | (Family: none) | |
| CN 104725366 A | 24 June 2015 | (Family: none) | |
| JP 2015-86179 A | 07 May 2015 | (Family: none) | |
| WO 2008/093639 A1 | 07 August 2008 | US 2010/0016396 A1<br>reference examples 194, 195, examples 103, 104, 131, 134, 169, 170, 176, 177, 191, 192, 213, 214, 216, 217, 226, 227<br>EP 2128138 A1 | |
| DE 4200742 A1 | 23 July 1992 | (Family: none) | |
| JP 2-96568 A | 09 April 1990 | (Family: none) | |
| JP 1-197471 A | 09 August 1989 | (Family: none) | |
| JP 63-183564 A | 28 July 1988 | US 4843068 A<br>claim 2, examples<br>EP 234045 A2<br>DE 3686649 A<br>BR 8606430 A<br>HU 43933 A<br>CA 1300137 A<br>AR 245934 A<br>KR 10-1989-0003850 B1<br>KR 10-1990-0002063 B1<br>CN 86108691 A<br>CN 1061321 A<br>AU 6692186 A | |
| JP 62-53969 A | 09 March 1987 | CS 631286 A<br>HU 43931 A<br>ES 2001627 A<br>KR 10-1987-0002081 A | |
| WO 2021/245087 A1 | 09 December 2021 | JP 2023-528891 A<br>US 2023/0278994 A1<br>EP 4161906 A1<br>BR 112022024394 A<br>CN 115803320 A | |
| US 3468884 A | 23 September 1969 | (Family: none) | |
| US 3574201 A | 06 April 1971 | GB 1286513 A<br>DE 1795410 A<br>FR 2019032 A<br>CH 513195 A<br>CH 515268 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022212727 A
- JP 2022514651 W
- WO 2021255071 A1
- WO 2016201168 A1
- WO 2017031325 A1
- JP UNEXAMINEDPATENTAPPLICATIONPUBLICATION A


### Non-patent literature cited in the description


- *CHEMICAL ABSTRACTS*, 943137-49-3